# EUROPEAN PATENT APPLICATION

(11) **EP 4 559 898 A1**
(43) Date of publication of application: **28.05.2025**
(21) Application number: 23462006.0
(22) Date of filing: 24.11.2023
(51) Int. Cl.: C07C 323/52, C07D 207/09, A61K 47/18, A61K 9/127

(54) **NOVEL IONIZABLE CATIONIC LIPIDS CONTAINING THIOETHER LINKAGE**

(71) Applicant: AldexChem Kft., 2030 Érd (HU)
(72) Inventor: Répási, József, 2030 Érd (HU); Szilvágyi, Gábor, 2017 Pócsmegyer (HU)
(74) Representative: Danubia Patent & Law Office LLC

(57) **Abstract**

The invention relates to novel ionizable amine lipids incorporating one or more sulphur atoms in the tail section. These lipids can be used in combination with other components to form lipid nanoparticles with oligonucleotides. The invention describes the synthesis of the lipids of formula (I), formation and characterization of nanoparticles and biological experiments demonstrating that the lipid nanoparticles prepared with these novel lipids can efficiently deliver their cargo (e.g. RNA, DNA, mRNA, siRNA, miRNA, pDNA, circular DNA, dsRNA, small biologically active molecules) into the cells.

## Description

### TECHNICAL FIELD

The present invention belongs to the field of biomedicine and drug delivery.

The invention relates to novel ionizable amine lipids incorporating one or more sulphur atoms in the tail section. These lipids can be used in combination with other components to form lipid nanoparticles with oligonucleotides. The invention describes the synthesis of the lipids of formula (I), formation and characterization of nanoparticles and biological experiments demonstrating that the lipid nanoparticles prepared with these novel lipids can efficiently deliver their cargo (e.g. RNA, DNA, mRNA, siRNA, miRNA, pDNA, circular DNA, dsRNA, small biologically active molecules) into the cells.

### BACKGROUND

There are many challenges associated with the delivery of nucleic acids-based agents e.g. messenger RNA, antisense oligonucleotides, ribozymes, plasmids and of small molecules etc. to trigger a desired response in a biological system. There is a need to design and synthesize novel second generation lipids (biodegradable ionizable amine lipids) in order to improve the delivery of biologically active agents, for example DNA, RNA, mRNA and various small molecules to cells. In the field of medicine those treatments are the most effective which can selectively and directly target affected cells or tissues with the appropriate active pharmaceutical agents. With this in mind, the efficiency of treatment shall be increased, and undesirable toxic side effects shall be avoided or reduced. Loaded lipid nanoparticles (LNPs), containing lipid components have been proven to be an effective carrier system which can be functionalized to protect and deliver the payload to its targeted site. It is to be emphasized that LNPs formed from ionizable cationic lipids also have proven to be outstanding lipid-based carriers in gene therapy.

LNPs also contain synthetic lipids which may be toxic to human cells and the cytotoxicity of synthetic lipids depends on certain motifs (e.g. type of tail, headgroup, linker) in the lipids relating to their biodegradability and path of their metabolism in the human body.

Incorporation of sulphur atom in the tail sections of the lipids is based on the following main considerations. The incorporated sulphur atom contributes to better biodegradability, fine-tuning of the physicochemical properties and addition points of diversification. Moreover, the thioether moiety gives a possibility to fine-tune the sensitivity to oxidation, and it could be a key element of e.g. Reactive Oxygen Species responsive drug delivery system.

There is a growing demand in the art of efficient, biodegradable, less toxic delivery platforms, since in the next decade the gene therapy is going to become part of the routine treatments.

The designed lipid structures according to the present invention are structurally different from the cationic lipid compounds know in the prior art (e.g. EP4122920A1 or US10166298B2) and have improved properties.

### DETAILED DESCRIPTION

In one embodiment, the invention provides an ionizable cationic lipid of formula (I) 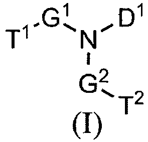
or its salt or stereoisomer,
wherein
   - G¹: is unsubstituted C₂-C₉ alkylene,
   -(CH₂)ₓ-CH=CH-(CH₂)_{y}-, wherein x is an integer selected from 1 to 6, y is an integer selected from 1 to 6, and the sum of x+y is an integer selected from 2 to 7, or
   -(CH₂)_{w}-X-(CH₂)_{z}-, wherein w is an integer selected from 1 to 7, z is an integer selected from 2 to 7, the sum of w+z is an integer selected from 3 to 8, wherein -(CH₂)_{z}- is attached to N, and X is selected from O, S;
   - T¹: is 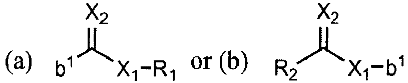 wherein
   b¹ is a bond to G¹,
   X₁ and X₂ are the same or different and each independently represents a or S,
   R₁ is linear C₁-C₃₂ alkyl, branched C₃-C₃₂ alkyl, in both cases containing one S at any position in the carbon chain with the proviso that the heteroatom is not in the alpha or omega position of the carbon chain, or
   R₁ is linear C₁-C₃₂ alkenyl, branched C₃-C₁₇ alkenyl in both cases containing one or more double bonds with the proviso that there is at least one -CH₂- group between the double bond and X₁ and in both cases containing one S at any position in the carbon chain with the proviso that the heteroatom is not in the alpha or omega position of the carbon chain and with the further proviso that there is at least one -CH₂- is between the heteroatom and the adjacent carbon atom of the double bond,
   R₂ is linear C₁-C₃₂ alkyl, branched C₃-C₃₂ alkyl, in both cases optionally containing one S at any position in the carbon chain with the proviso that the heteroatom is not in the alpha or omega position of the carbon chain, or
   R₂ is linear C₁-C₃₂ alkenyl, branched C₃-C₃₂ alkenyl containing one or more double bonds with the proviso that there is at least one -CH₂- group between the double bond and the carbon linking
   X₁ and X₂ and in both cases containing one S at any position in the carbon chain with the proviso that the heteroatom is not in the alpha or omega position of the carbon chain and with the further proviso that there is at least one -CH₂- is between the heteroatom and the adjacent carbon atom of the double bond;
   - G²: is defined as G¹ above, where G¹ and G² may be identical or different;
   - T²: is defined as T¹ above, wherein b¹ is a bond to G², and where T¹ and T² may be identical or different,
   - D¹: is selected from 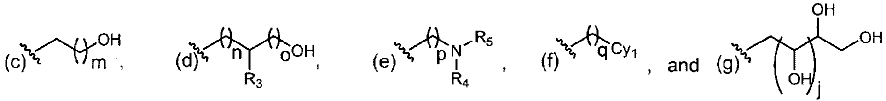 wherein
   R₃ is C₁-C₆ alkyl, cyclopentyl, cyclohexyl, hydroxyl, hydroxymethyl, hydroxyethyl, phenyl, benzyl, 4-hydroxy benzyl, 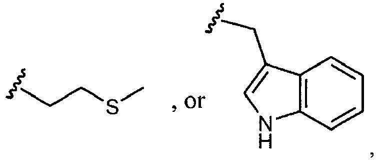
   R₄ and R₅ are independently selected from H and C₁-C₆ alkyl,
   m is an integer selected from 1 to 6,
   n is an integer selected from 0 to 6,
   o is an integer selected from 0 to 6,
   p is an integer selected from 2 to 6,
   q is an integer selected from 0 to 6,
   j is an integer selected from 1 to 4,
   Cy₁ is a C₃-C₆ cycloalkyl optionally substituted by one or more -OH, or
   Cy₁ is a pyranose, furanose ring, which can be linked via any of its OH group, wherein the pyranose or furanose ring is optionally substituted by a -NH-CO-CH₃ group, adenine, guanine, uracil, cytosine, thymine, a mono- or oligosaccharide, or by a 4-, 5-, 6- or 7-membered heterocyclic ring containing 1, 2, 3 or 4 heteroatoms selected from N, O or S; or
   Cy₁ is a 4-, 5-, 6- or 7-membered heterocyclic ring containing 1, 2, 3 or 4 heteroatoms selected from O, N or S, wherein the heterocyclic ring is optionally substituted by R₆, wherein
   R₆ is C₁-C₆ alkyl, an arginine containing peptide, a pyranose or furanose ring attached by any of their ring-carbon atoms to the 4-, 5-, 6- or 7-membered heterocyclic ring, wherein the pyranose or furanose ring is optionally substituted by a -NH-CO-CH₃ group, adenine, guanine, uracil, cytosine, thymine or a mono- or oligosaccharide, or
   R₆ is a group selected from (c), (d), (e) or (g), wherein, m, n, o, p, j, R₃, R₄ and R₅ are as defined above, or
   R₆ is a 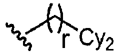 group wherein r is an integer selected from 1 to 4 and Cy₂ is a 4-, 5-, 6- or 7-membered heterocyclic ring containing 1, 2, 3 or 4 heteroatoms selected from O, N and S, optionally substituted by C₁-C₆ alkyl.

By unsubstituted C₂-C₉ alkylene we mean ethylene, propylene, butylene, pentylene, hexylene, heptylene, octylene, nonylene.

By C₁-C₆ alkyl group we mean a linear or branched alkyl group containing 1 to 6 carbon atoms, e.g. methyl, ethyl, propyl, butyl, pentyl, hexyl, isopropyl, isobutyl, *tert*-butyl, neopentyl, isopentyl, neohexyl or isohexyl.

By linear C₁-C₃₂ alkyl, branched C₃-C₃₂ alkyl, in both cases the chain containing one S at any position in the carbon chain with the proviso that the heteroatom is not in the alpha or omega position of the carbon chain, we mean a linear C₁-C₃₂ alkyl or branched C₃-C₃₂ alkyl group containing 1-32 or 3-32 carbon atoms respectively, wherein one -CH₂- of the alkyl chain, that is not in the terminal position at any side of the chain, is replaced by S atom, e.g. one of the following groups in case of R₁: 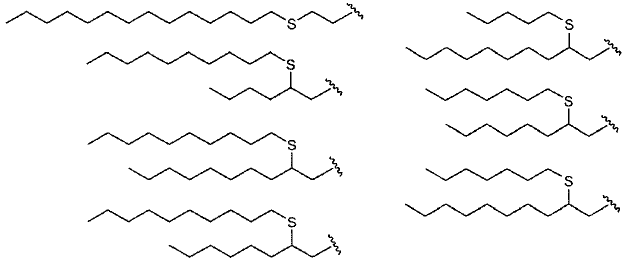 e.g. one of the following groups in case of R₂:

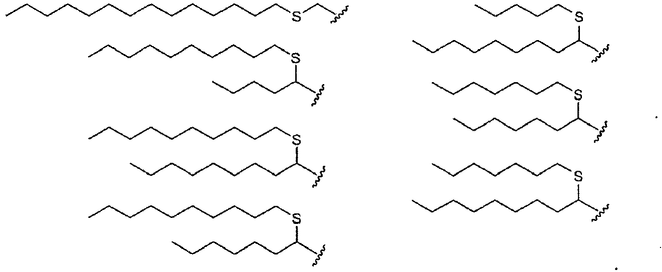

By linear C₁-C₃₂ alkenyl, branched C₃-C₃₂ alkenyl in both cases containing one or more double bonds with the proviso that there is at least one -CH₂- group between the double bond and X₁ or the carbon linking X₁ and X₂, and in both cases containing one S at any position in the carbon chain with the proviso that the heteroatom is not in the alpha or omega position of the carbon chain and at least one -CH₂- is between the heteroatom and the adjacent carbon atom of the double bond, we mean a mono- or polyunsaturated linear or branched C₃-C₃₂ alkenyl group, in which there is a -CH₂- or 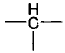 group in the terminal position of the alkyl chain that is the link to the other part of the compound, e.g. one of the following groups in case of R₁: 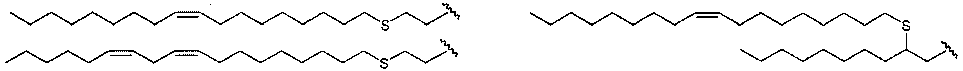 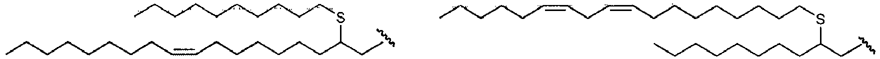 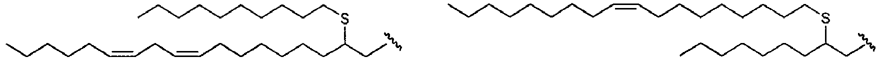 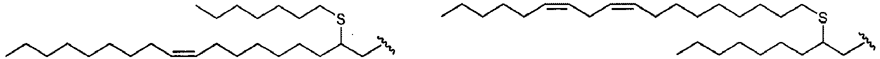 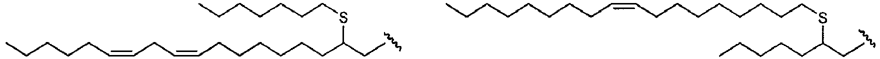 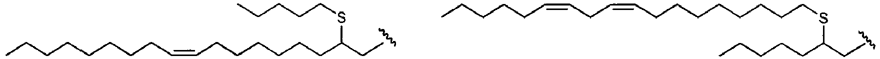 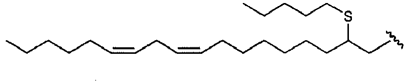 e.g. one of the following groups in case of R₂:

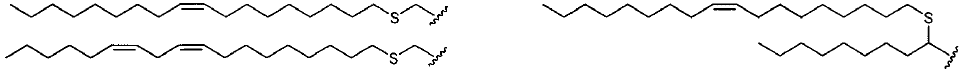

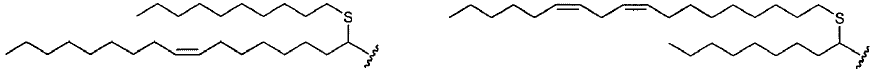

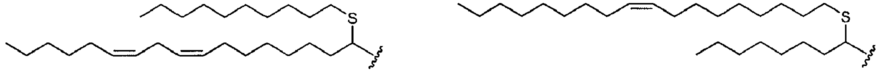

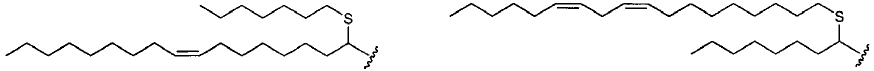

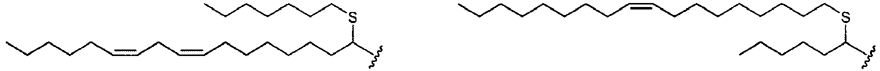

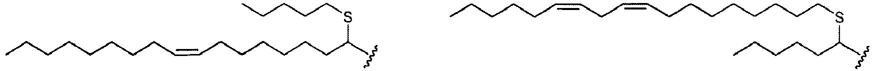

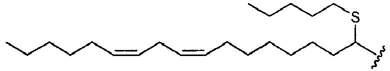

By C₃-C₆ cycloalkyl we mean cyclopropyl, cyclobutyl, cyclopentyl, cyclohexyl.

By -O-C(O)-C₁-C₆ alkyl we mean C₁-C₆ acylate group e.g.: isobutanoate, 2,2-dimethylpropanoate.

By a 4-, 5-, 6- or 7-membered heterocyclic ring containing 1, 2, 3 or 4 heteroatoms selected from O, N or S mean an aromatic ring or an unsaturated, partly saturated or fully saturated heterocyclic ring, e.g.: azetine, azetidine, pyrrolidine, oxazolidine, piperidine, piperazine, morpholine, pyrrole, imidazole, pyrazole, 1,2,3-triazole, 1,2,4-triazole, tetrazole, pyridine, pyrimidine, pyridazine, pyrazine, 1,2,4-triazine, 1,3,5-triazine, 1,2,4,5 tetrazine, oxazole, isoxazole, thiazole, isothiazole, 1,2-oxazine, 1,3-oxazine, 1,4-oxazine, 1,2-thiazine, 1,3-thiazine, 1,4-thiazine, azepane, azepine, 1,2-diazepane, 1,3-diazepane, 1,4-diazepane, 1,2-diazepine, 1,3-diazepine, 1,4-diazepine, 1,2-oxazepane, 1,3-oxazepane, 1,4-oxazepane, 1,2-oxazepine, 1,3-oxazepine, 1,4-oxazepine, 1,2-thiazepane, 1,3-thiazepane, 1,4-thiazepane, 1,2-thiazepine, 1,3-thiazepine, or 1,4-thiazepine ring, preferably pyrrolidine, imidazole, 1,2,3-triazol, piperidine, piperazine, morpholine, furane, thiophene, pyrane, thiopyran, oxetane, tetrahydrofuran, or dioxane.

By furanose we mean all stereoisomers of ribofuranose, deoxyribofuranose, xylofuranose, fructofuranose, glucofuranose, galactofuranose, mannofuranose, allofuranose, arabinofuranose, or altrofuranose, which can be also protected with e.g. acetyl, benzyl, benzoyl, isopropylidene or benzylidene groups. For example: 2,3,5,6-tetra-O-acetyl-galactofuranose.

By pyranose we mean all stereoisomers of glucopyranose, galactopyranose, mannopyranose, allopyranose, fructopyranose, arabinopyranose or altropyranose, which can also be protected with e.g. acetyl, benzyl, benzoyl, isopropylidene, or benzylidene groups. For example: 2,3,4,6-tetra-O-acetyl-glucopyranose.

By mono-, oligosaccharide we mean monosaccharide as defined above for furanose and pyranose and we mean by oligosaccharide e.g. sucrose, lactose, maltose, isomaltulose, cellobiose, trehalose.

By arginine containing peptide we mean a peptide with 6 amino acids or less comprising at least one arginine residue. The peptide is linked by its C or N terminus.

By salts of the compounds of formula (I) we mean salts of the compounds of formula (I) with inorganic or organic acids. Preferred salts are those with pharmaceutically acceptable acids. The salts are e.g. chloride, sulfate, phosphate, formate, acetate, fumarate, maleate, oxalate, citrate or tartrate. The salts formed during purification or isolation are also subject of the invention.

By stereoisomers we mean optical and geometric isomers. The compounds of formula (I) may contain one or more asymmetric carbon atoms thus they can exist in the form of optical isomers, enantiomers or diastereomers. The compounds of formula (I) may contain double bounds and the groups attached to the double bond can have different (cis or trans) confirmations, e.g. cis or trans fatty acid moieties.

A narrower group of compounds of formula (I) is, wherein G¹ and G² are the same or different and each is independently linear C₄-C₉ alkylene, preferably C₅-C₉ alkylene, or, alternatively, each is independently linear C₅, C₆, C₇, or C₉ alkylene.

One embodiment of the invention is a compound of formula (I), wherein T¹ is (a).

One embodiment of the invention is a compound of formula (I), wherein T¹ is (b).

In a particular embodiment G¹-T¹ and G²-T² are identical.

In an other particular embodiment G¹-T¹ and G²-T² are different.

One particular embodiment of the invention is a compound of formula (I), wherein T¹ and/or T² has one of the following structures:

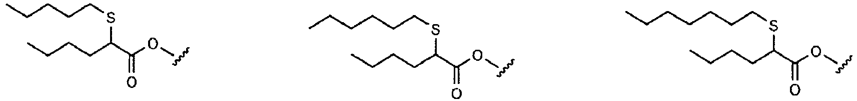

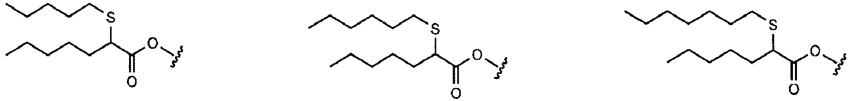

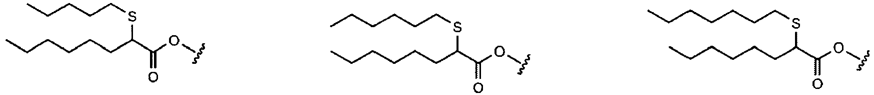

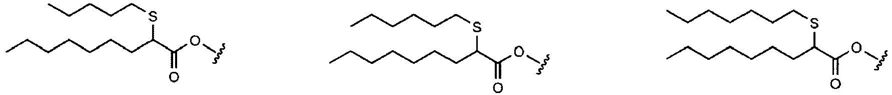

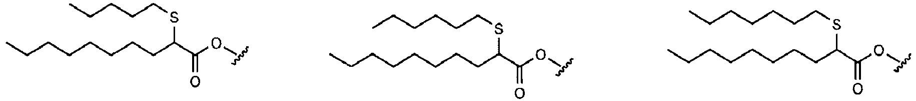

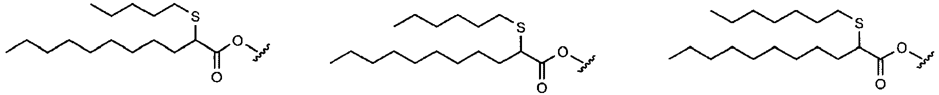

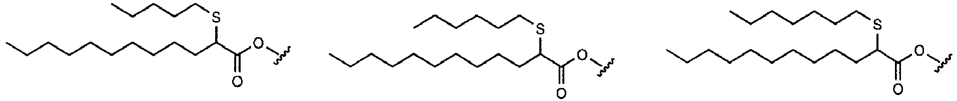

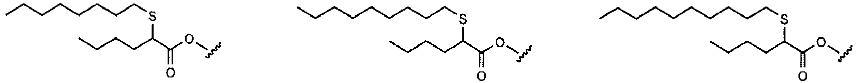

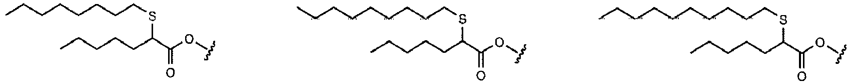

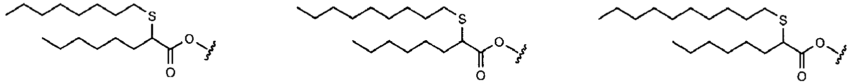

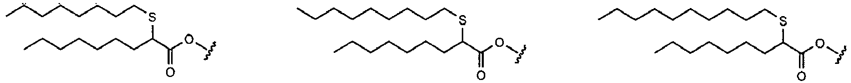

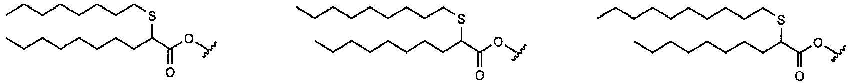

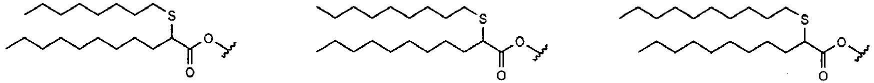

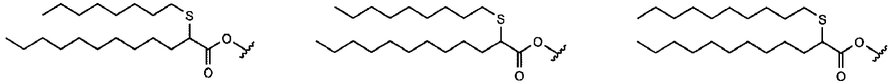

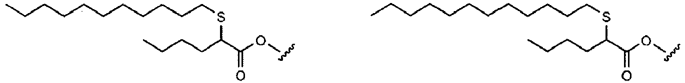

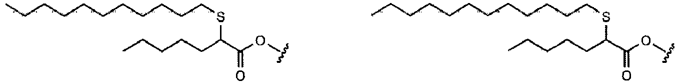

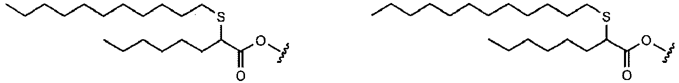

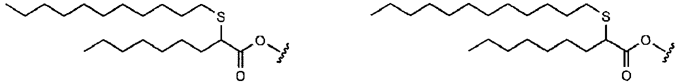

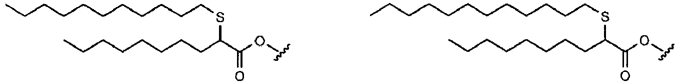

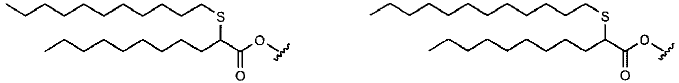

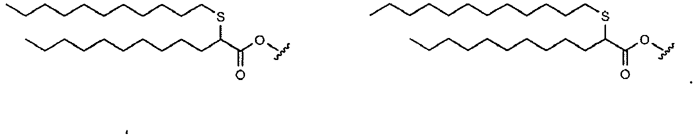

One embodiment of the invention is a compound of formula (I), wherein T¹ and/or T² has one of the following structures:

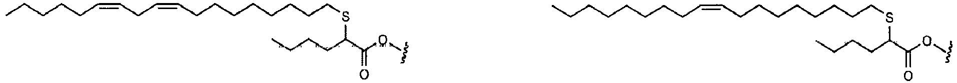

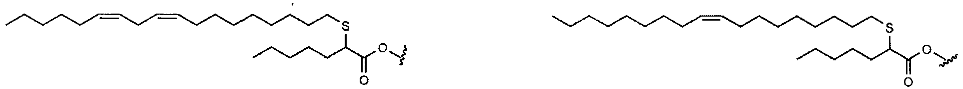

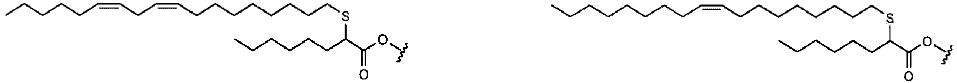

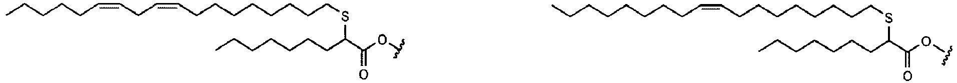

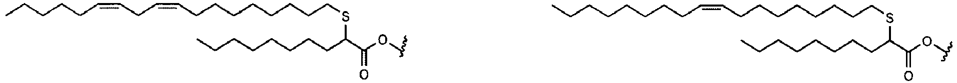

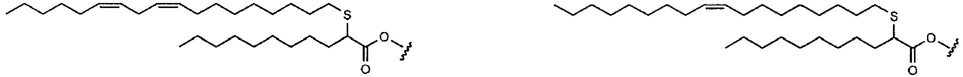

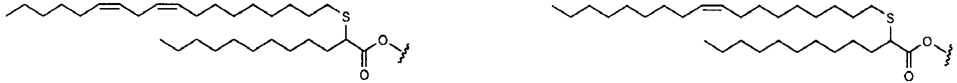

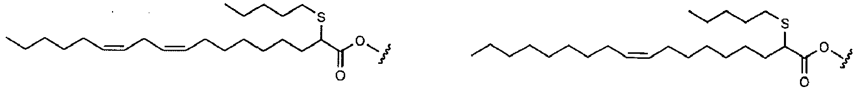

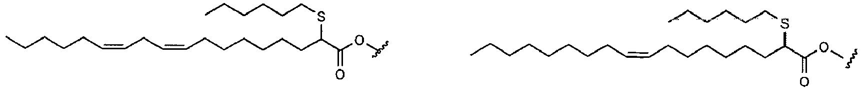

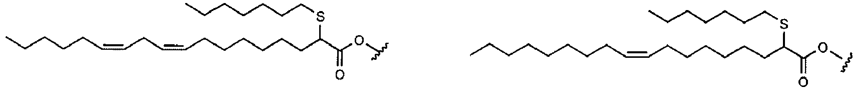

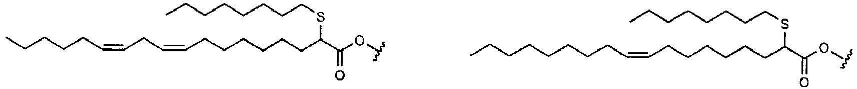

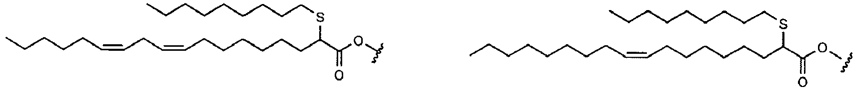

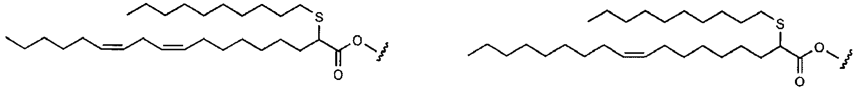

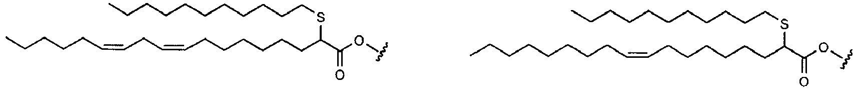

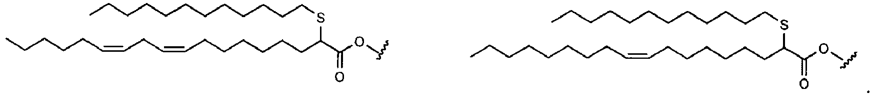

One embodiment of the invention is a compound of formula (I), wherein T¹ and/or T² has one of the following structures:

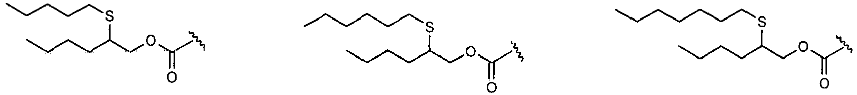

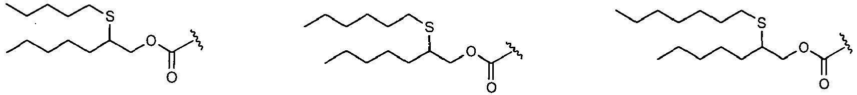

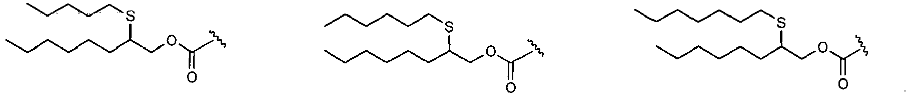

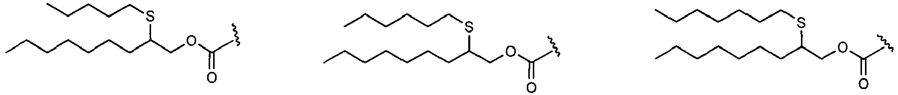

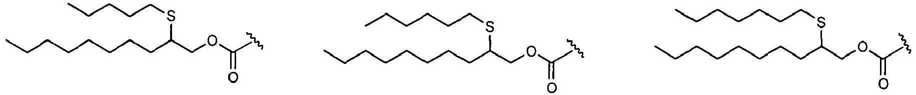

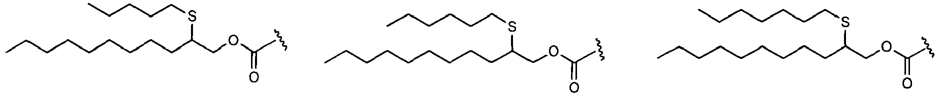

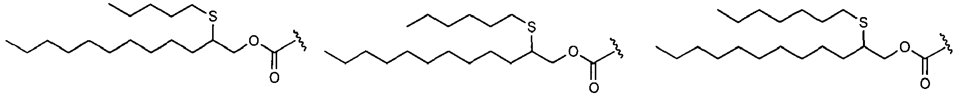

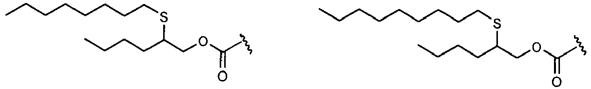

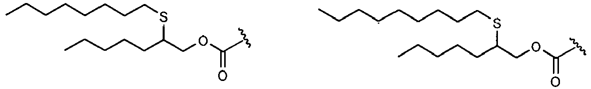

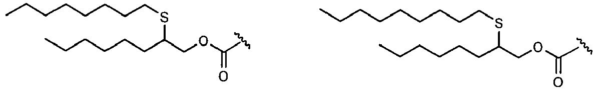

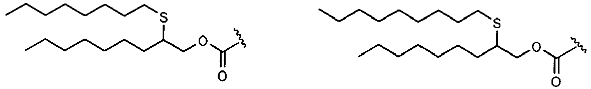

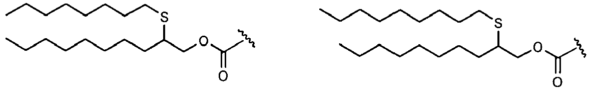

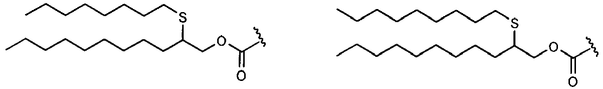

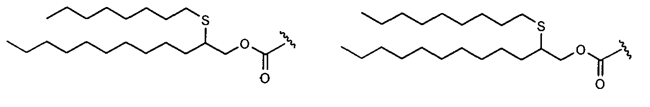

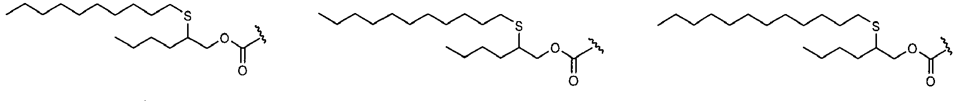

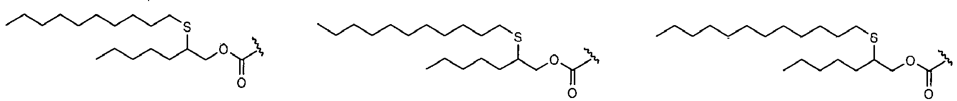

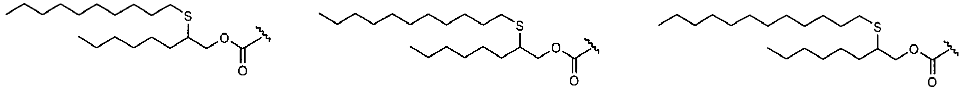

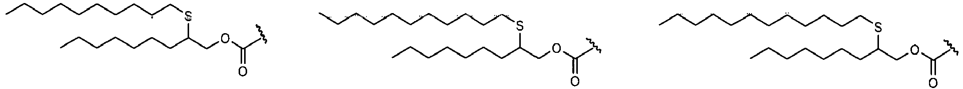

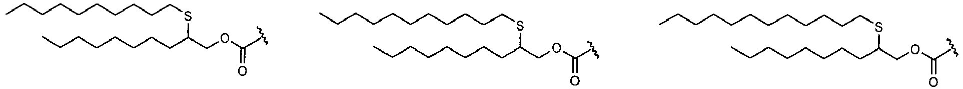

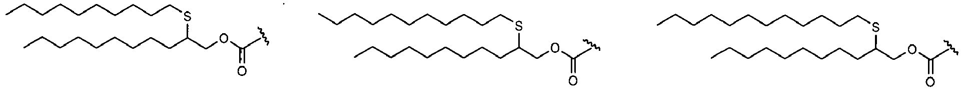

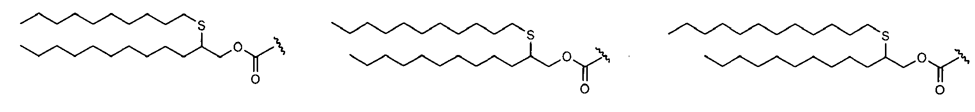

One embodiment of the invention is a compound of formula (I), wherein T¹ and/or T² has one of the following structures:

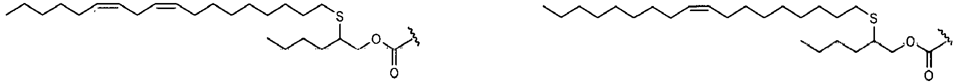

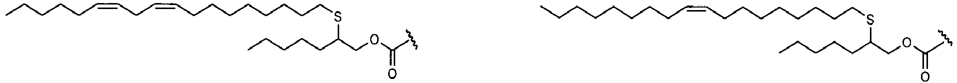

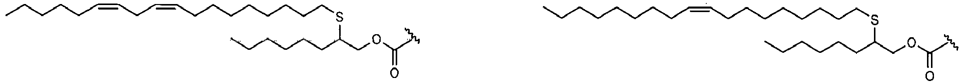

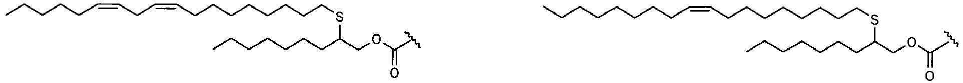

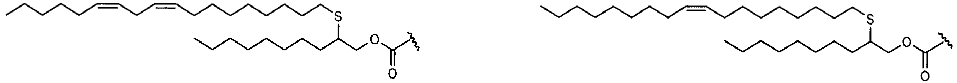

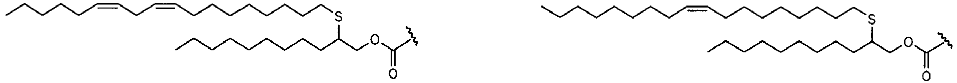

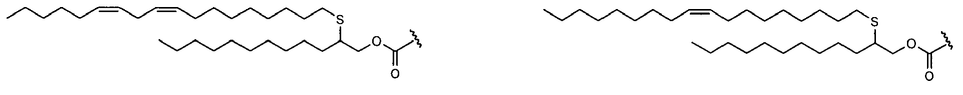

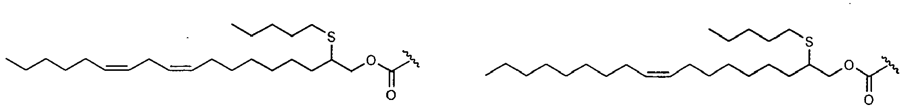

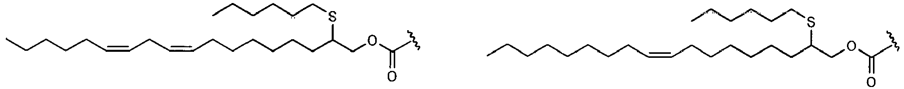

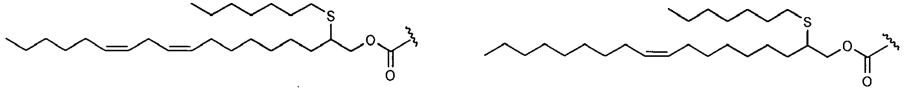

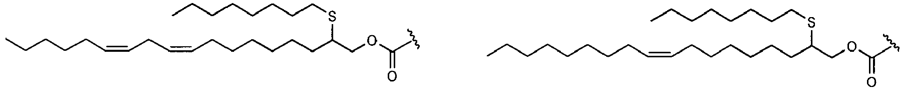

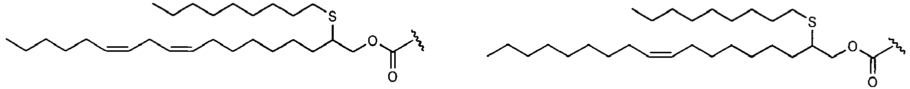

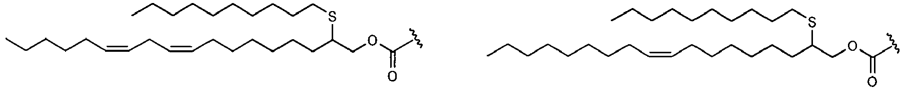

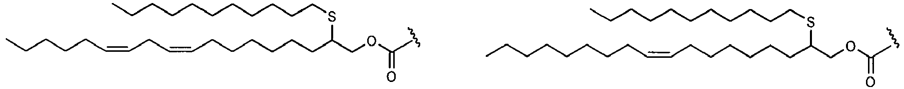

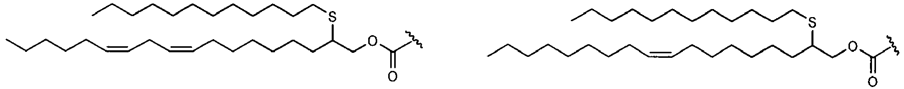

One embodiment of the invention is a compound of formula (I), wherein T¹ and/or T² has one of the following structures:

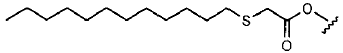

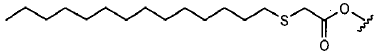

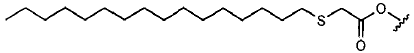

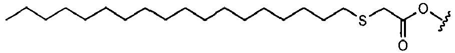

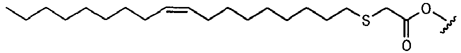

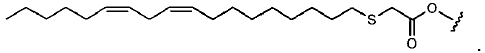

One embodiment of the invention is a compound of formula (I), wherein T¹ and/or T² has one of the following structures:

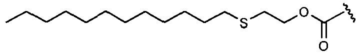

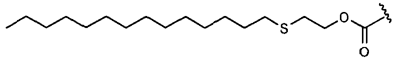

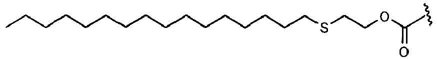

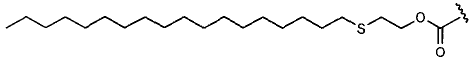

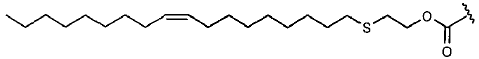

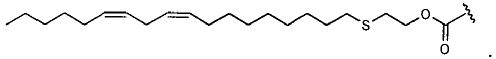

One embodiment of the invention is a compound of formula (I), wherein T¹ and/or T² has one of the following structures:

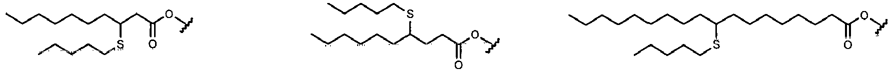

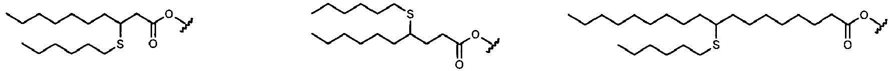

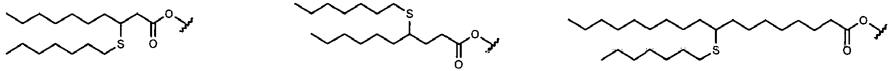

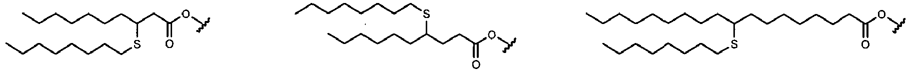

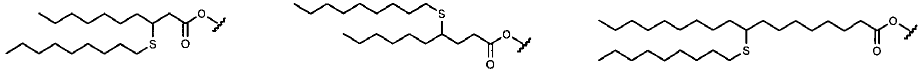

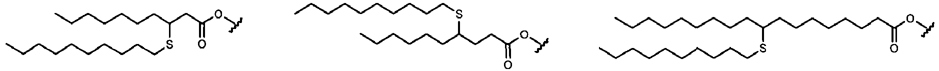

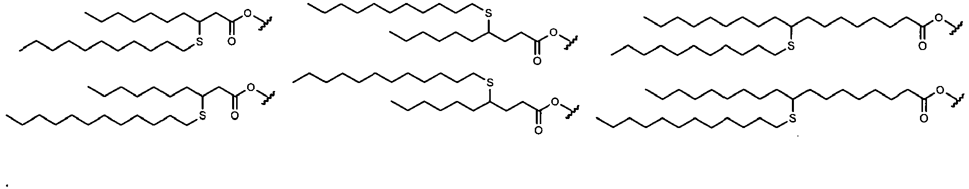

One embodiment of the invention is a compound of formula (I), wherein T¹ and/or T² has one of the following structures:

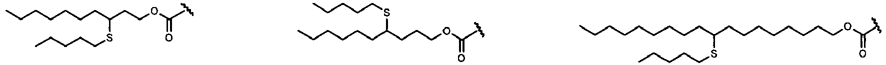

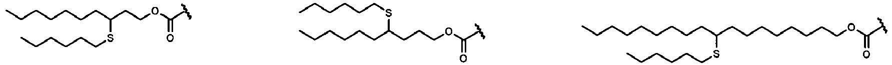

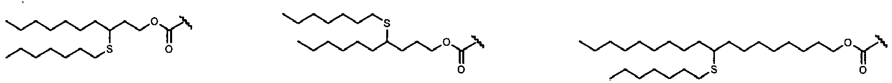

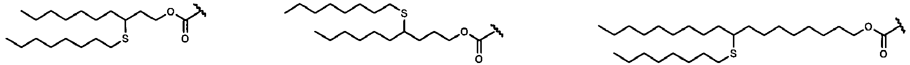

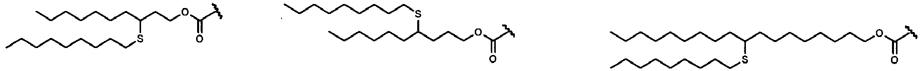

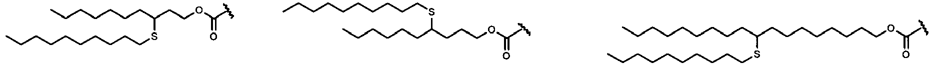

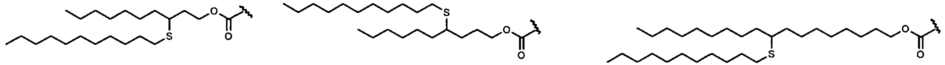

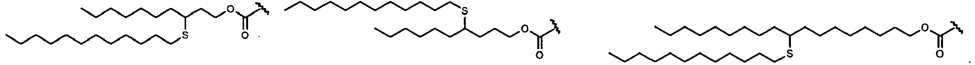

One embodiment of the invention is a compound of formula (I), wherein D¹ has one of the following structures:

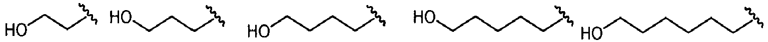

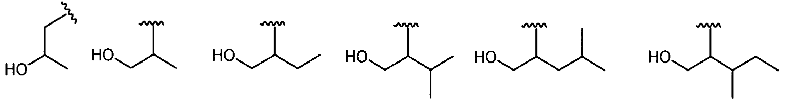

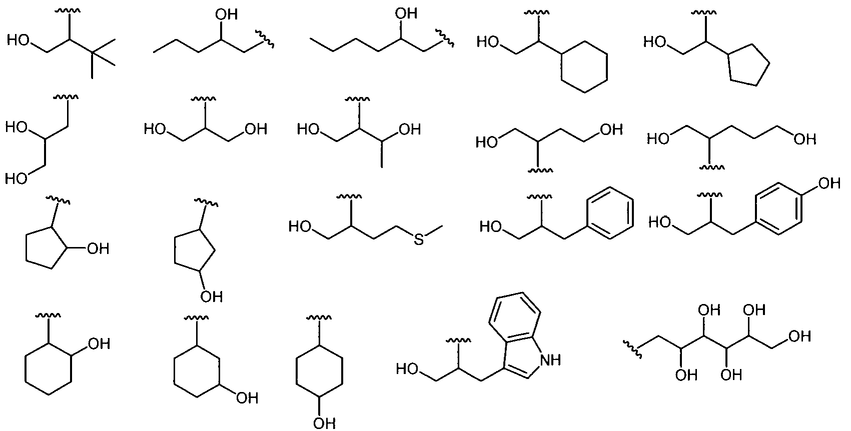

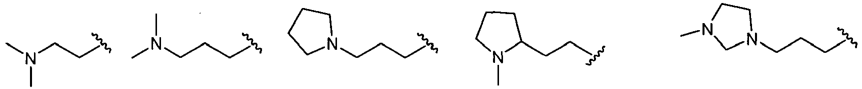

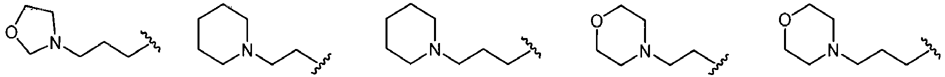

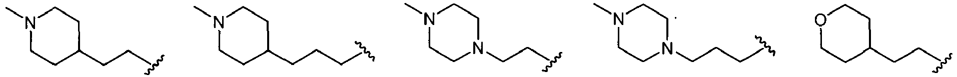

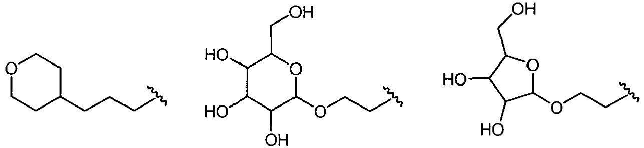

A particular embodiment within this group is, wherein D¹ has one of the following structures:

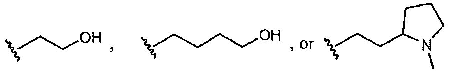

One embodiment of the invention is a compound of formula (I), wherein D¹ has one of the following structures: 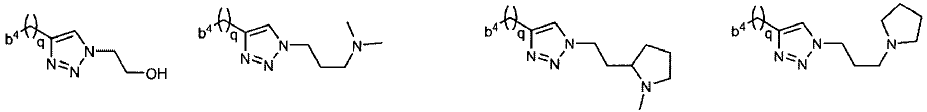 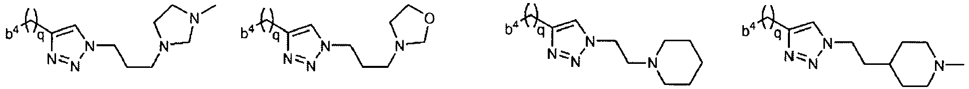 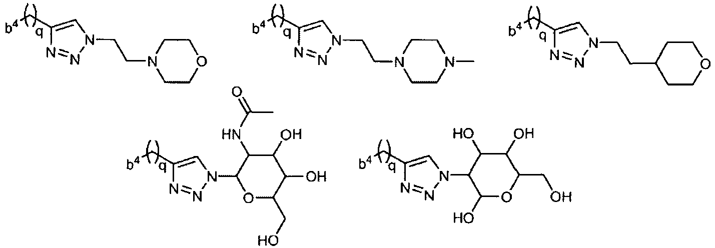 wherein q is an integer selected from 1 to 4 and b⁴ is a bond to the nitrogen.

A further subject of the invention is the use of the compounds of formula (I) as defined above for the preparation of lipid nanoparticle (LNP).

The invention also provides a lipid nanoparticle (LNP) comprising compound of formula (I) as defined above and a therapeutic agent (e.g. RNA, DNA, mRNA, siRNA, pDNA, circular DNA, dsRNA, or small biologically active molecules).

The building blocks used for the preparation of the compounds of the formula (I) and the compounds of formula (I) can be prepared according to the following general reaction schema.

The chemical reactions used for the preparation of the corresponding building blocks are well documented in the literature and a skilled person can perform these reactions without undue burden.

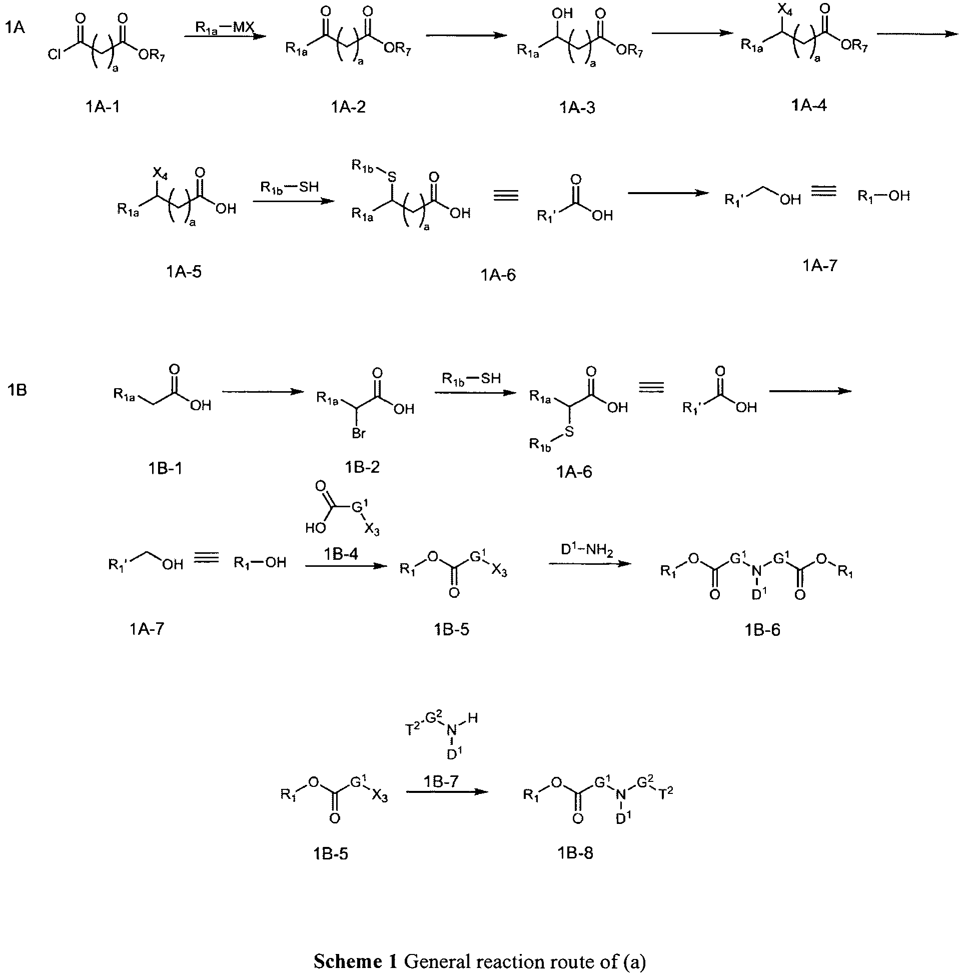

**Scheme 1A and 1B** illustrate the general reaction route of formula (I) having T¹ and T² which are selected from (a). The synthesis can be started from the appropriate dicarboxylic acid monoester chloride (R₇: Me-, Et-, tBu-, a means an integer from 1 to 10) in that cases wherein sulphur in T¹ can be attached to any carbon atom from β to ω-1 carbon atom (Scheme 1A). The dicarboxylic acid monoester chloride 1A-1 is reacted with R₁ₐ-MX Grignard reagent (R₁ₐ: alkyl, alkenyl, M: Zn, Mn) yielding the desired oxo-carboxylic acid ester 1A-2 (transmetallation with e.g. ZnCl₂ or MnCl₂). In the following step, the oxo is reduced to hydroxy 1A-3 (e.g. NaBH₄ ) and which is transformed to an appropriate leaving group (X₄: Br, OMs, OTs, OTf) in 1A-4 (e.g. MsCl and TEA). Then, the ester 1A-4 is hydrolysed to the corresponding carboxylic acid 1A-5 which is followed by alkylation/alkenylation with R_{1b}-SH compound (R_{1b}: alkyl, alkenyl) yielding 1A-6 (e.g. KOtBu). Finally, the compound 1A-6 is reduced to yield 1A-7 (e.g. LiAlH₄) as a building block of the lipids. The Scheme 1B illustrates that special case, wherein sulphur is attached to α carbon atom. The synthesis can be started from a carboxylic acid 1B-1 (R₁ₐ: alkyl, alkenyl) which is reacted with bromine yielding α-brominated compound 1B-2. In the next step, the compound 1B-2 is reacted with R_{2b}-SH compound (R_{1b}: alkyl, alkenyl) in order to get the alkylation/alkenylation (e.g. KOtBu) compound 1-A6 and subsequently 1-A6 is transformed to 1A-7 as mentioned above.

Thereafter, the synthesis of the corresponding lipid can be achieved with an esterification reaction between compound 1A-7 and 1B-4 yielding compound 1B-5 (e.g. DCC). Finally 1B-5 is reacted with an amine yielding a symmetric lipid 1B-6 (e.g. cat. KI, K₂CO₃). In addition, asymmetric lipid can be synthesized if 1B-5 can be reacted with a monoalkylated compound 1B-7 which leads to 1B-8.

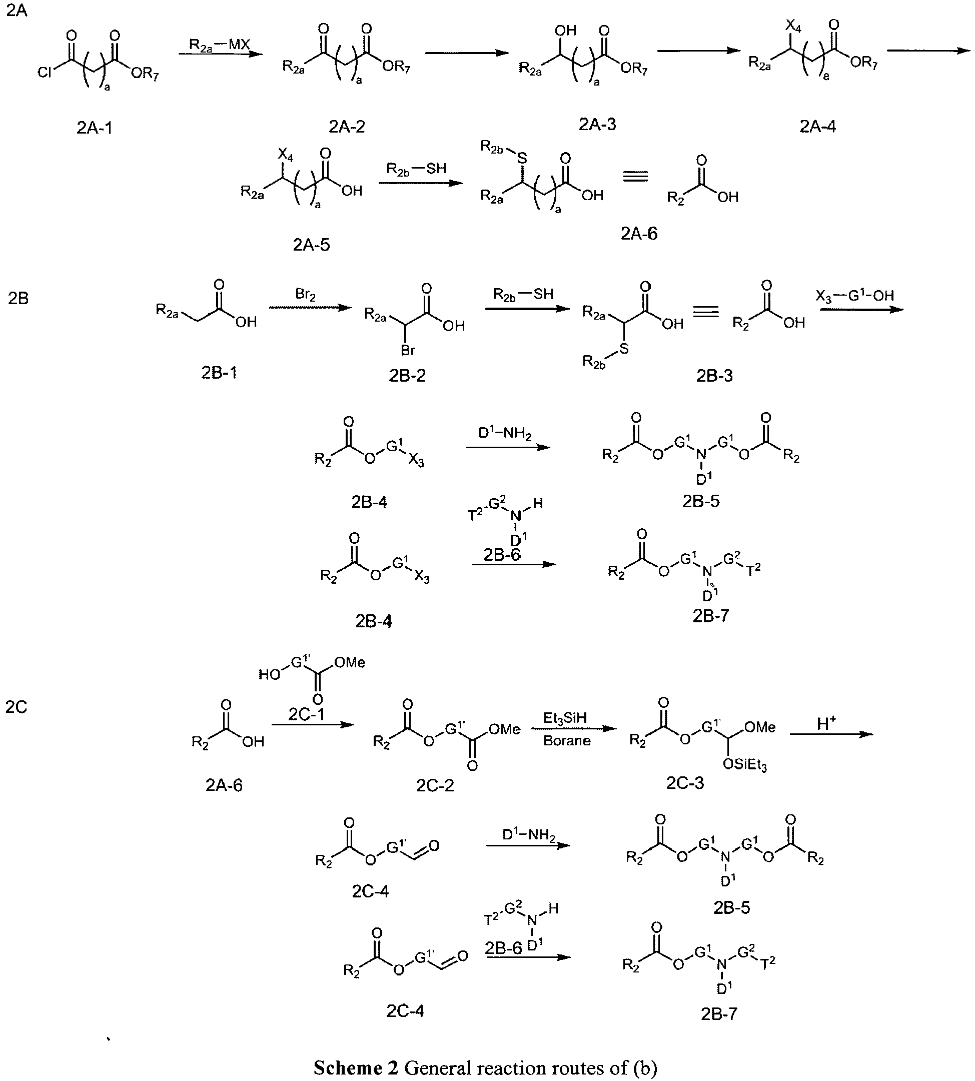

**Scheme 2A and 2B** illustrate the general reaction route of formula (I) having T₁ and T₂ which are selected from (b). The synthesis can be started from the appropriate dicarboxylic acid monoester chloride 2A-1 (R₇: Me-, Et-, tBu-, a means an integer from1 to10) in that cases wherein sulphur in the T¹ can be attached to any carbon atom from β to ω-1 carbon atom (Scheme 2A). The dicarboxylic acid monoester chloride 2A-1 is reacted with R₂ₐ-MX Grignard reagent (R₂ₐ: alkyl, alkenyl, M: Zn, Mn) yielding the desired oxo-carboxylic acid ester 2A-2 (transmetallation with e.g. ZnCl₂ or MnCl₂). In the following step, the oxo is reduced to hydroxy 2A-3 (e.g. NaBH₄) and which is transformed to an appropriate leaving group (X₄: Br, OMs, OTs, OTf) in 2A-4 (e.g. MsCl, TEA). Then, the ester 2A-4 is hydrolyzed to the corresponding carboxylic acid 2A-5 which is followed by alkylation/alkenylation with R_{1b}-SH compound (R_{1b}: alkyl, alkenyl) yielding 2A-6 (e.g. KOtBu) as a building block of the lipids. The Scheme 2B illustrates that special case, wherein sulphur is attached to α carbon atom. The synthesis can be started from a carboxylic acid 2B-1 (R₂ₐ: alkyl, alkenyl) which is reacted with bromine yielding α-brominated compound 2B-2. In the next step, the compound 2B-2 is reacted with R_{2b}-SH compound (R_{2b}: alkyl, alkenyl) in order to get the alkylation/alkenylation compound 2B-3 (e.g. KOtBu) as a key building block.

Thereafter, the synthesis of the corresponding lipid can be achieved with an esterification reaction 2B-3 yielding compound 2B-4 (e.g. DCC). Finally 2B-4 is reacted with an amine yielding a symmetric lipid 2B-5 (e.g. cat. KI, K₂CO₃). In addition, asymmetric lipid can be synthesized if 2B-4 can be reacted a monoalkylated compound 2B-6 which leads to 2B-7.

According to another approach, the synthesis of the corresponding lipid can be achieved with an esterification reaction between compound 2A-6 and 2C-1 yielding compound 2C-2 (e.g. DCC). In the next step, the compound 2C-2 is reduced with triethylsilane in the presence of a proprietary borane catalyst [borane catalysts are disclosed in WO2022/129966] yielding mixed silyl acetal 2C-3 and subsequently it is hydrolysed to the corresponding aldehyde 2C-4 (e.g. HCl in AcOH/H₂O). Finally, the 2C-4 is reacted with an amine in reductive alkylation conditions in order to get a symmetric lipids 2B-5 (e.g. AcOH, NaHB(OAc)₃). In addition, asymmetric lipid can be synthesized in that case if 2C-4 can be reacted a monoalkylated amine compound 2B-6 which leads to 2B-7.

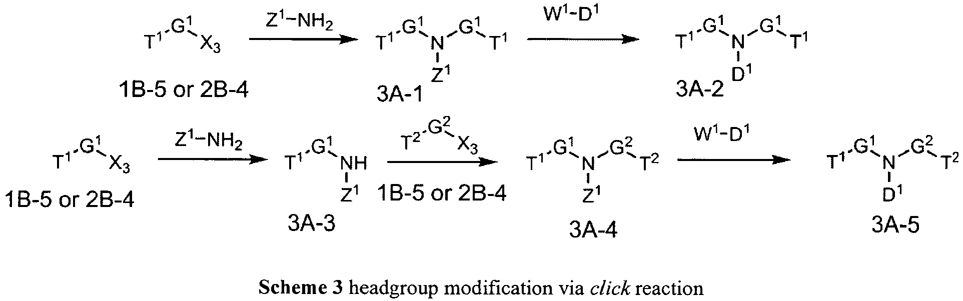

**Scheme 3** illustrates methods to make headgroup (D¹) modified lipids via *click* chemistry, wherein T¹-G¹, T²-G², D¹ are defined above, X₃ is halogen or pseudohalogen, W¹ is N₃ or ethynyl depending on the meaning of Z¹. The 1B-5 or 2B-4 intermediate is reacted with Z¹-NH₂ having the appropriate functional group for the following click reaction step. Finally 3A-1 is reacted with W¹-D¹ under click reaction condition yielding a symmetric lipid 3A-2 (e.g. azidosugar, CuBr, PMDT in DMF). In addition, asymmetric lipid 3A-5 can be synthesized based on mentioned above (Scheme 1-2).

### Chromatography methods for the purification of the lipids and intermediates

### Purification Method A

Eluent A: EtOAc, eluent B: petroleum ether +1% Et₃N; full gradient A:B 0:100 -> 100:0. Sample loading ~200 mg; column size: 25 g of SiO₂. Column conditioned with ~400 mL of B prior loading the sample.

### Purification Method B

Eluent A: DCM:MeOH:aq. NH₃ 80:20:1, eluent B: DCM; full gradient A:B 0:100 -> 100:0. Sample loading ~200 mg; column size: 25 g of SiO₂. Column conditioned with -400 mL of A and then with ~400 mL of B prior loading the sample.

### General Procedure A

A MW reaction vial was charged with 2.0 eq. aldehyde, 1.0 eq. amine, 0.5 eq. acetic acid and abs. toluene (1.0 mmol aldehyde/3.1 ml of abs. toluene). After 1 h stirring at room temperature, 3.0 eq. sodium triacetoxyborohydride was added and the resulting mixture was stirred for overnight. Then the reaction mixture was diluted with ethyl acetate and NaHCO₃ solution and stirred vigorously for 5 minutes and the phases were separated. The organic phase was washed with brine, separated and dried over Na₂SO₄, filtered and concentrated.

### General Procedure B

A MW reaction vial was charged with ω-bromo ester (eq. specified in the example) and 1.0 eq. amine, abs. acetonitrile (1.0 mmol ω-bromo ester/0.95 ml of abs. acetonitrile) and abs. cyclopentyl methyl ether (1.0 mmol ω-bromo ester/0.31 ml of abs. cyclopentyl methyl ether). To the stirred reaction mixture K₂CO₃ (3.0 eq.) and KI (0.2 eq.) were added and purged with argon and sealed. The resulting mixture was stirred for 18 hours at 63°C. Then the reaction mixture was cooled down to room temperature and partitioned between ethyl acetate and brine and stirred vigorously for 15 minutes. The organic phase was separated and dried over Na₂SO₄, filtered and concentrated.

### Examples

The invention is illustrated by the following examples. These examples illustrate, but do not limit the invention. For all of the examples, standard work-up and purification methods known to those skilled in the art can be utilized.

### Example 1

### Synthesis of ((4-hydroxybutyl)azanediyl)bis(hexane-6,1-diyl) bis(2-(decylthio)hexanoate)

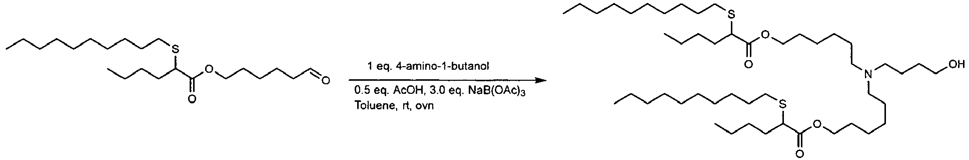

((4-hydroxybutyl)azanediyl)bis(hexane-6,1-diyl)bis(2-(decylthio)hexanoate) was prepared from 6-oxohexyl 2-(decylthio)hexanoate (1006.3 mg, 2.603 mmol, 2.0 eq.) and 4-amino-1-butanol (116 mg, 0.1.302 mmol, 1.0 eq.) according to **General Procedure A**. The crude product was purified by flash column chromatography according to **Purification Method A** to obtain the pure product as a colourless oil, 340 mg, 0.409 mmol, 32 % yield.

¹H NMR (500 MHz, Benzene-d₆) δ 4.19-4.06 (m, 4H), δ 3.61 (t, 2H), δ 3.31 (t, 2H), δ 2.78-2.68 (m, 2H), δ 2.67-2.57 (m, 2H), δ 2.33-2.23 (m, 6H), δ 2.09-1.99 (m, 2H), δ 1.83-1.73 (m, 2H), δ 1.73-1.12 (m, 60H), δ 0.94-0.90 (t, 6H), δ 0.84-0.81 (t, 6H); LC ESI MS [M+H⁺]: 830.732 m/z

### Example 2

### Synthesis of ((4-hydroxybutyl)azanediyl)bis(hexane-6,1-diyl) bis(2-(pentylthio)decanoate)

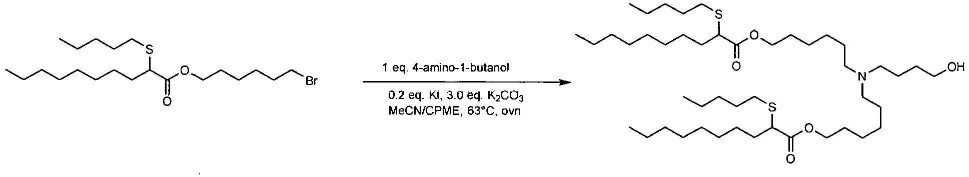

((4-hydroxybutyl)azanediyl)bis(hexane-6,1-diyl) bis(2-(pentylthio)decanoate) was prepared from 6-bromohexyl 2-(pentylthio)decanoate (1177.8 mg, 2.692 mmol, 2.0 eq.) and 4-amino-1-butanol (120 mg, 0.1.346 mmol, 1.0 eq.) according to **General Procedure B.** The crude product was purified by flash column chromatography according to **Purification Method A** to obtain the pure product as a colourless oil, 166 mg, 0.207 mmol, 15 % yield.
¹H NMR (500 MHz, CDCl₃) δ 4.13-4.09 (m, 4H), δ 3.54 (t, 2H), δ 3.20 (t, 2H), δ 2.63-2.51 (m, 4H), δ 2.44-2.40 (m, 6H), δ 1.89-1.80 (m, 2H), δ 1.68-1.12 (m, 58H), δ 0.91-0.85 (m, 12H); TOF MS ES⁺[M+H⁺]: 802.6423 m/z

### Example 3

### Synthesis of ((4-hydroxybutyl)azanediyl)bis(octane-8,1-diyl) bis(2-(decylthio)hexanoate)

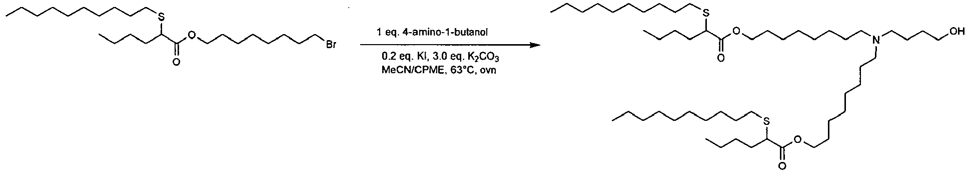

((4-hydroxybutyl)azanediyl)bis(octane-8,1-diyl) bis(2-(decylthio)hexanoate) was prepared from 8-bromooctyl 2-(decylthio)hexanoate (941.6 mg, 1.963 mmol, 2.5 eq.) and 4-amino-1-butanol (70 mg, 0.785 mmol, 1.0 eq.) according to **General Procedure B**. The crude product was purified by flash column chromatography according to **Purification Method** A to obtain the pure product as a colourless oil, 115 mg, 0.130 mmol, 16 % yield.
¹H NMR (500 MHz, Benzene-d₆) δ 4.18-4.08 (m, 4H), δ 3.63 (t, 2H), δ 3.33 (t, 2H), δ 2.78-2.71 (m, 2H), δ 2.67-2.60 (m, 2H), δ 2.37-2.33 (m, 4H), δ 2.30-2.27 (m, 2H) δ 2.09-2.00 (m, 2H), δ 1.83-1.74 (m, 2H), δ 1.67-1.18 (m, 68H), δ 0.94-0.91 (t, 6H), δ 0.85-0.81 (t, 6H); LC ESI MS [M+H⁺]: 886.839 m/z

### Example 4

### Synthesis of ((2-hydroxyethyl)azanediyl)bis(hexane-6,1-diyl) bis(2-(decylthio)hexanoate)

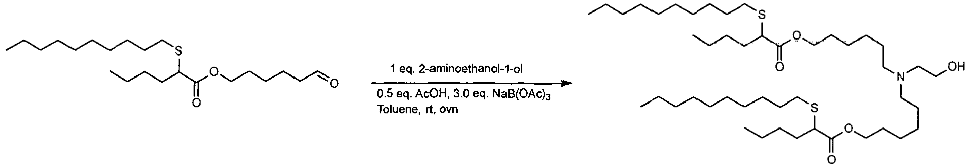

((2-hydroxyethyl)azanediyl)bis(hexane-6,1-diyl) bis(2-(decylthio)hexanoate) was prepared from 6-oxohexyl 2-(decylthio)hexanoate (468.39 mg, 1.211 mmol, 2.0 eq.) and 2-aminoethan-1-ol (37 mg, 0.605 mmol, 1.0 eq.) according to **General Procedure A**. The crude product was purified by flash column chromatography according to **Purification Method B** to obtain the pure product as a colourless oil, 197 mg, 0.245 mmol, 40 % yield.
¹H NMR (500 MHz, Benzene-d₆) δ 4.17-4.07 (m, 4H), δ 3.50 (t, 2H), δ 3.32 (t, 2H), δ 2.78-2.69 (m, 2H), δ 2.68-2.59 (m, 2H), δ 2.35 (t, 2H), δ 2.27-2.24 (m, 4H) δ 2.09-1.98 (m, 2H), δ 1.83-1.73 (m, 2H), δ 1.68-1.10 (m, 56H), δ 0.94-0.90 (t, 6H), δ 0.84-0.81 (t, 6H); LC ESI MS [M+H⁺]: 802.769 m/z

### Example 5

### Synthesis of ((2-(1-methylpyrrolidin-2-yl)ethyl)azanediyl)bis(hexane-6,1-diyl) bis(2-(decylthio)hexanoate)

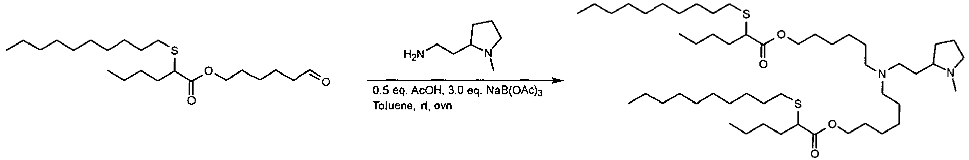

((2-(1-methylpyrrolidin-2-yl)ethyl)azanediyl)bis(hexane-6,1-diyl) bis(2-(decylthio)hexanoate) was prepared from 6-oxohexyl 2-(decylthio)hexanoate (467.96 mg, 1.210 mmol, 2.0 eq.) and 2-(1-methylpyrrolidin-2-yl)ethan-1-amine (77.6 mg, 0.605 mmol, 1.0 eq.) according to **General Procedure A**. The crude product was purified by flash column chromatography according to **Purification Method A** to obtain the pure product as a colourless oil, 187 mg, 0.215 mmol, 35 % yield.
¹H NMR (500 MHz, Benzene-d₆) δ 4.06-3.97 (m, 4H), δ 3.11 (t, 2H), δ 2.98-2.94 (m, 1H), δ 2.54-2.41 (m, 4H), δ 2.36-2.24 (m, 5H), δ 2.21 (s, 3H), δ 2.05-1.99 (m, 1H) δ 1.95-1.12 (m, 68H), δ 0.81-0.75 (m, 12H); LC ESI MS [M+H⁺]: 869.818 m/z

### Example 6

### Synthesis of ((4-hydroxybutyl)azanediyl)bis(hexane-6,1-diyl) bis(2-(heptylthio)decanoate)

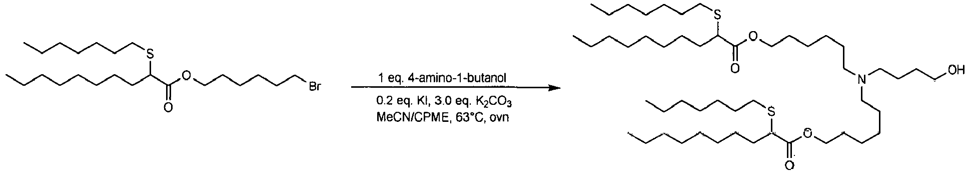

((4-hydroxybutyl)azanediyl)bis(hexane-6,1-diyl) bis(2-(heptylthio)decanoate) was prepared from 6-bromohexyl 2-(heptylthio)decanoate (1253.3 mg, 2.692 mmol, 2.0 eq.) and 4-amino-1-butanol (120 mg, 1.346 mmol, 1.0 eq.) according to **General Procedure B.** The crude product was purified by flash column chromatography according to **Purification Method A** to obtain the pure product as a colourless oil, 110 mg, 0.128 mmol, 10 % yield.
¹H NMR (500 MHz, Benzene-d₆) δ 4.17-4.07 (m, 4H), δ 3.61 (t, 2H), δ 3.35 (t, 2H), δ 2.78-2.69 (m, 2H), δ 2.67-2.60 (m, 2H), δ 2.31-2.23 (m, 6H), δ 2.11-2.01 (m, 2H), δ 1.85-1.77 (m, 2H), δ 1.65-1.16 (m, 64H), δ 0.92-0.87 (m, 12H); TOF MS ES⁺[M+H⁺]: 858.7054 m/z

### Example 7

### Synthesis of ((4-hydroxybutyl)azanediyl)bis(hexane-6,1-diyl) bis(2-(decylthio)decanoate)

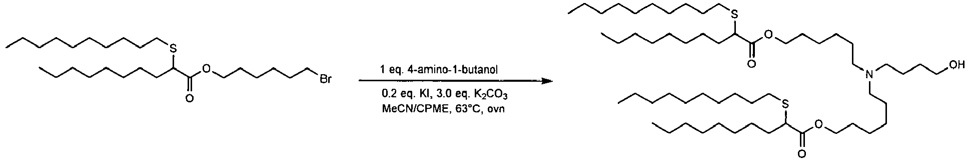

((4-hydroxybutyl)azanediyl)bis(hexane-6,1-diyl) bis(2-(decylthio)decanoate) was prepared from 6-bromohexyl 2-(decylthio)decanoate (1456.2 mg, 2.962 mmol, 2.2 eq.) and 4-amino-1-butanol (120 mg, 1.346 mmol, 1.0 eq.) according to **General Procedure B.** The crude product was purified by flash column chromatography according to **Purification Method A** to obtain the pure product as a colourless oil, 640 mg, 0.679 mmol, 50 % yield.
¹H NMR (500 MHz, Benzene-d₆) δ 4.18-4.10 (m, 4H), δ 3.61 (t, 2H), δ 3.35 (t, 2H), δ 2.78-2.71 (m, 2H), δ 2.68-2.61 (m, 2H), δ 2.30-2.23 (m, 6H), δ 2.11-2.02 (m, 2H), δ 1.86-1.76 (m, 2H), δ 1.66-1.16 (m, 76H), δ 0.94-0.89 (m, 12H); TOF MS ES⁺[M+H⁺]: 942.7987 m/z

### Example 8

### Synthesis of ((4-hydroxybutyl)azanediyl)bis(hexane-6,1-diyl) bis(2-(decylthio)octanoate)

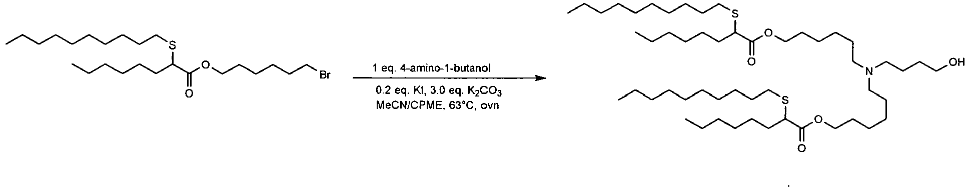

((4-hydroxybutyl)azanediyl)bis(hexane-6,1-diyl) bis(2-(decylthio)octanoate) was prepared from 6-bromohexyl 2-(decylthio)octanoate (941.6 mg, 1.963 mmol, 2.5 eq.) and 4-amino-1-butanol (70 mg, 0.785 mmol, 1.0 eq.) according to **General Procedure B**. The crude product was purified by flash column chromatography according to **Purification Method A** to obtain the pure product as a colourless oil, 430 mg, 0.485 mmol, 62 % yield.
¹H NMR (500 MHz, Benzene-d₆) δ 4.18-4.08 (m, 4H), δ 3.62 (t, 2H), δ 3.34 (t, 2H), δ 2.78-2.71 (m, 2H), δ 2.68-2.61 (m, 2H), δ 2.30-2.23 (m, 6H), δ 2.11-2.00 (m, 2H), δ 1.84-1.76 (m, 2H), δ 1.65-1.16 (m, 68H), δ 0.94-0.85 (m, 12H); TOF MS ES⁺[M+H⁺]: 886.7386 m/z

### Example 9

### Synthesis of ((4-hydroxybutyl)azanediyl)bis(hexane-6,1-diyl) bis(2-(heptylthio)octanoate)

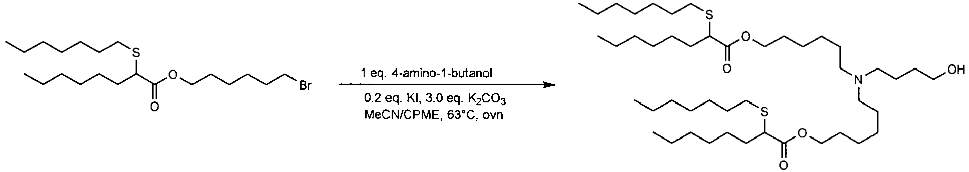

((4-hydroxybutyl)azanediyl)bis(hexane-6,1-diyl) bis(2-(heptylthio)octanoate) was prepared from 6-bromohexyl 2-(heptylthio)octanoate (1296.0 mg, 2.962 mmol, 2.2 eq.) and 4-amino-1-butanol (120 mg, 1.346 mmol, 1.0 eq.) according to **General Procedure B.** The crude product was purified by flash column chromatography according to **Purification Method A** to obtain the pure product as a colourless oil, 147 mg, 0.183 mmol, 14 % yield.
¹H NMR (500 MHz, Benzene-d₆) δ 4.19-4.11 (m, 4H), δ 3.62 (t, 2H), δ 3.34 (t, 2H), δ 2.77-2.70 (m, 2H), δ 2.66-2.59 (m, 2H), δ 2.30-2.23 (m, 6H), δ 2.10-2.00 (m, 2H), δ 1.84-1.75 (m, 2H), δ 1.64-1.13 (m, 56H), δ 0.90-0.85 (m, 12H); TOF MS ES⁺[M+H⁺]: 802.6429 m/z

### Example 10

### Synthesis of ((4-hydroxybutyl)azanediyl)bis(hexane-6,1-diyl) bis(2-(tetradecylthio)acetate)

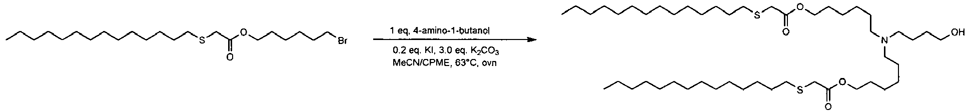

((4-hydroxybutyl)azanediyl)bis(hexane-6,1-diyl) bis(2-(tetradecylthio)acetate) was prepared from 6-bromohexyl 2-(tetradecylthio)acetate (1337.4 mg, 2.962 mmol, 2.2 eq.) and 4-amino-1-butanol (120 mg, 1.346 mmol, 1.0 eq.) according to **General Procedure B**. The crude product was purified by flash column chromatography according to **Purification Method A** to obtain the pure product as a colourless oil, 690 mg, 0.831 mmol, 62 % yield.
¹H NMR (500 MHz, Benzene-d₆) δ 4.03 (t, 4H), δ 3.61 (t, 2H), δ 3.00 (s, 4H), δ 2.58 (t, 4H), δ 2.29-2.23 (m, 6H), δ 1.59-1.11 (m, 68H), δ 0.93-0.90 (m, 6H); LC ESI MS [M+H⁺]: 830.777 m/z

### LNP formulation and dialysis

### Preparation:

1. If needed, prepare stock solutions by solving known amounts of lipid powder into an appropriate solvent. The recommended solvent is ethanol and the recommended concentration is typically in the range 10-50 mM. Higher concentrations are preferred to allow flexibility on mRNA-LNP concentration.
2. Remove the lipid stock solutions from the -20°C freezer. For long-term storage, -80°C is used.
3. Check the vials for precipitation, DOPE (CAS# 4004-05-1) (20 mM) and DSPE-PEG (CAS# 474922-26-4) (5mM) may need a 5-30 minute incubation step at 37°C with intermittent vortexing to resuspend all contents. Vials that are significantly precipitated (from repeated freeze-thawing) may not be recoverable. As a last resort, lipids may be heated to 50°C to resuspend, time should be kept as short as possible to prevent oxidation or other modifying or degradative processes.
4. Mix the lipids by vortexing for 2 minutes.
5. Cool the lipids stock solutions to room temperature by incubating on the bench, optionally protected from light. Cooling the solution prevent excessive evaporation of ethanol or similar solvents upon opening.
6. Meanwhile, thaw the RNA samples, including the latest SecNLuc control, by inserting the frozen tube in a room temperature aluminium block.
7. Prepare for each LNP-formulation with identical mRNA component, an RNA master mix according to Table 1 below in a fresh tube. Use of a master mix allows more precise comparison of lipid formulations relative to each other. Split the master mix over a number of fresh tubes equal to the number of samples + controls. Each tube contains 375 µl of mRNA in 10 mM Citrate pH 4.0.
8. Prepare for each LNP formulation with identical lipid composition a Lipid master mix according to Table 1 below in a fresh tube, exclude the lipid of interest. Use of a master mix allows more precise comparison of the novel ionizable lipid relative to each other. Split the master mix over a number of fresh tubes equal to the number of samples + controls. Each tube contains 65.5 µl of lipid master mix.
9. Add the ionizable lipid of interest to the equally divide lipid master mix. Mix well by pipetting up and down. Label the tube according to the ionizable lipid used.
10. Power on the L1 FM (L1 Formulation Machine uses 2 high-speed syringe pumps to mix lipids in organic solvents with mRNA in acidic aqeuous solution to generate well-defined LNPs. The microfluidic mixing chip involves a dean-type structure performing the high-efficiency mixing at a total flow rate (TFR) of around 12ml/min at a 3:1 mixing ratio) and allow it to do a calibration of motor positions.

**Table 1: Lipid formulation solution**

| Name | Ionizable lipid | | Cationic lipid | | Helper lipid | | Core lipid | | Shield lipid | | EtOH | mRNA | Citrate 100mM | RFW* |
|---|---|---|---|---|---|---|---|---|---|---|---|---|---|---|
| Sample | 59.2 µL | Tested lipid (10mM) | 4.8 µL | DOTAP (CAS# 132172-61-3) (25mM) | 4.7 | DOPE (30mM) | 19.7 µL | Resveratrol (CAS# 501-36-0) (25mM) | 1.25 µL | DSPE-PEG (5mM) | 35 µL | 41 µL | 37.5 µL | 297 µL |
| Master mix for 23 samples | | | 120 µL | | 117.5 µL | | 492.5 µL | | 31.25 µL | | 1500 µL | 1025 µL | 937.5 µL | 7425 µL |

| | | | | | | | | | | | | | | |
|---|---|---|---|---|---|---|---|---|---|---|---|---|---|---|
| * Ribonuclease free water | | | | | | | | | | | | | | |

### Rinsing and pre-wetting:

11. Place a fresh microfluidic cartridge (COC-plastic) into the loading slot of the L1 FM.
12. Load the 'Receiving station' of the L1 FM with 2 ml Eppendorf tubes.
13. Fill one 'Rinse syringe' (A) with 1 ml RNAse-free H₂O and the other (B) with 1 ml 99% EtOH from the designated 'Wash' stock solutions
14. Attach the syringes in the correct orientation, the H₂O-filled syringe on the left and the EtOH-filled syringe on the right
15. Rinse the chip by pressing 'Button 2' (100 µg), hold the chip in place and check the movement of the syringes while the machine is working
16. Restart the machine for the next formulation.
17. Discard the flow-through

### Formulation:

18. Fill a fresh syringe (C) through a blunt 18G stainless steel needle with the previously prepared RNA mixture of step 6. The volume should be approximately 85% of total formulation volume + 25 µl (dead-volume). Make sure to remove all air bubbles by tapping.
19. Fill a fresh syringe (D) through a blunt 18G stainless steel needle with the Lipid master mix. The volume should be approximately 35% of total formulation volume + 25 µl (dead-volume). Again, Make sure to remove all bubbles.
20. Attach the syringes in the correct orientation, syringe A on the left and syringe B on the right
21. Load the 'Receiving station' with appropriate sized collection containers (2 ml Eppendorf tubes).
22. Press the microfluidic chip to the back of the machine with your thumb and hold, press the 'Button 1' (40 µg). The L1 FM pushes the total volume from the syringes in a perfect 3:1 (mRNA aqueous: lipid in EtOH) ratio through the microfluidic mixer chip with a total flow-rate of 12 ml/ml (Total flow rate (TFR)).
23. Remove the sample tube from the 'Receiving station' and label the tube accordingly.
24. Reset the machine to prepare with the next formulation.

### Dialysis:

25. Transfer the individual formulations to 100 kda MWCO, SpectraPor Biotech dialysis tubing, and close with designated clamps.
26. Submerge the dialysis bags in beakers filled with 1x Phosphate-buffered saline (PBS buffer, pH 7.4, Gibco cat# 202212-019), stir at 230 RPM at room temperature.
27. Refresh the 1x PBS buffer pH 7.4 after 2 hours of incubation at room temperature, while stirring.
28. Refresh the buffer and incubate for 2 more hours at room temperature, while stirring.
29. Remove the dialysis bag from the dialysis setup and extract the LNP samples with a sterile pipette tip.
30. Dilute the samples to 1 ml with sterile 1x PBS and measure the Zeta potential, Size and polydispersity index (PDI) using the DLS machine.

### Size and size distribution measurements with dynamic light scattering

Turn on the DLS (Dynamic Light Scattering) machine 30 minutes before use. This allows the laser to warm up and the sample station to equilibrate to the set working temperature (usually room temperature).
1. Optionally; if producing large volume in discrete batches (i.e., processed in multiple dialysis cassettes), pool the individual formulations for each of the unique constructs.
2. Unpack the folded capillary cells (DTS1070), use one sample per construct.
3. Flush the cuvette by holding it upside down (injection ports below) and inject 1 ml H₂O in one of the ports. This will dispense the fluid through the cell and out of the other port.
4. Repeat this step with a syringe filled with 1 ml air, this will dry the excess of water that might remain in the cell after flushing.
5. Turn the cuvette so that the injection ports are on the top.
6. Dilute 10 µl of dialyzed LNP formulation in 690 µl of 1x PBS buffer, pH 7.4.
7. Add the sample to the cell by using a clear syringe, do not exceed the 'Fill, Max' line on the cuvette.
8. Select the 'Size & Zeta' option in the 'Method builder' of the 'ZS Explorer' software, and measure the samples to determine the size, PDI and Zeta potential.

### Encapsulation efficiency

### Solutions required:

1. Preparation of sample stock solutions: In the top row of the 96-well plate (Row A in the plate), add 297 µL of TE buffer pH 7.4 to a single well for each sample plus a single well for a PBS blank using a multichannel pipette.
TE buffer preparation:

| 500 ml TE buffer | Supplier/ cat# | |
|---|---|---|
| Tris-HCL (1M) pH 8.0 | Invitrogen ref: AM9856 | 5.0 ml |
| EDTA (0,5M) | Invitrogen ref:AM9261 | 1.0 ml |
| RFW | Invitrogen ref:1097-035 | 494 ml |

2. Add 3 µL of sample (at 0.5mg/ml) to these wells for a final volume of 300 µL. Add 3 µL of PBS to the blank well. Pipette to mix. This is your stock solution for each sample. The final RNA concentration of these stock solutions should be approximately 4-7 µg/mL.
3. mRNA-LNP sample setup:
a. Add 50 µL of TE buffer pH 7.5 to the two wells directly below each sample (Rows B and C in the plate).
b. Add 50 µL of sample stock solution from Row A into the wells in Rows B and C (this assay is run in duplicate. All liquid handling should be done using a multichannel pipette).
c. Add 50 µL of Triton buffer to the wells in Rows D and E (in the plate) below each sample.
d. Add 50 µL of sample stock solution from Row A into the wells in Rows D and E.
4. RiboGreen RNA standard curve setup: Dilute the RNA standard to produce an RNA stock at a final concentration of 20 µg/mL in TE buffer pH 7.4. The final volume should be 150 µL. Set up a standard curve (in duplicate) as using the RNA stock (20 µg/mL siRNA), TE buffer pH 7.5, and Triton X-100 buffer according the Table 2 below.
Triton X-100 buffer preparation:

| 500 ml Triton X-100 buffer | Supplier/ cat# | Amount |
|---|---|---|
| Tris-HCL (1M) pH 8.0 | Invitrogen ref: AM9856 | 5.0 ml |
| EDTA (0,5M) | Invitrogen ref:AM9261 | 1.0 ml |
| Triton X-100 | Arcos organics ref: AC327371000 | 5.0 ml |
| RFW | Invitrogen ref: 1097-035 | 489 ml |

**Table 2: RiboGreen RNA standard curve preparation**

| Final mRNA concentration | RNA stock (µl) | TE buffer (µl) | Triton buffer (µl) | Total volume (µl) |
|---|---|---|---|---|
| 2.5 | 25 | 25 | 50 | 100 |
| 1 | 10 | 40 | 50 | 100 |
| 0.5 | 5 | 45 | 50 | 100 |
| 0.25 | 2.5 | 47.5 | 50 | 100 |
| 0.1 | 1 | 49 | 50 | 100 |

5. Once samples and standard curve are plated, incubate the plate at 37°C for 10 min to lyse mRNA-LNP in the presence of Triton X-100 buffer.
6. Meanwhile, prepare RiboGreen Solution: Sum the total number of sample wells and standard curve wells. Add three to this number, and multiply the total by 100. This is the total volume, in µL, of RiboGreen Solution needed for this assay. In a 15 mL RNAse-free Falcon tube, dilute the RiboGreen Reagent 1:100 into TE buffer pH 7.5 to the total volume calculated.
7. Addition of RiboGreen Solution and sample readings: Remove a 96-well plate from 37°C incubator; let it cool to room temperature as RiboGreen binding is somewhat temperature dependent. Add 100 µL of RiboGreen Solution to each well. Pop any air bubbles with a needle. Read using fluorescent plate reader (excitation = 480, emission = 525).
8. Sample analysis: Use the data generated by the RiboGreen standard curve to calculate the concentration of mRNA.

### Transfection efficiency with Luciferase-mRNA

### Control transfection preparation:

1. Prepare mRNA solution by adding 100 ng mRNA (SecNLuc, #P009024, RiboPro) to 5 µl Opti-MEM
   - multiply by number of wells, include different concentrations and add 10% extra
   - a standard set-up we use is 100 ng - 50 ng - 10 ng mRNA/well in triplicate, which would need 528 ng mRNA (includes 10% extra)

### Transfection:

2. (If applicable) Prepare different concentrations in Opti-MEM (for instance 100/50/10 ng).
3. Add 10 µl to each well and mix carefully by pipetting.
4. Mix by swirling the plate horizontally at modest speed. Reverse in direction 3 times.
5. Incubate for 24 hours (incubator).

### Nano-Glo Assay

### Background:

This protocol is applied to measure luciferase activity for secreted NanoLuc and is based on the protocol by Promega (Nano-Glo^{®} Luciferase Assay System #N1110-1150). The protocol is designed for a 384-wells plate, but volumes can be changed accordingly for other plates.

### Procedure:

1. Plate cells (HeLa) in 96-well plate, grow until 80% confluent and transfect with SecNanoLuc mRNA and incubate for the desired period (24h).
2. After incubation, the translated protein is accumulated in the medium. Transfer medium to a new plate or tubes .
3. Transfer 12 µl of each sample to a black-walled, clear-bottom 384-well plate
   Black-walled plates are important to prevent shine-through of luminescence from other wells; alternatively, multiple wells between samples can be left empty.
4. Set-up plate reader
5. Prepare the desired amount of Nano-Gio^{®} Luciferase Assay Reagent by combining one volume of Nano-Glo^{®} Luciferase Assay Substrate with 50 volumes of Nano-Glo^{®} Luciferase Assay Buffer:
   a. composition: 100 mM MES pH 6; 1 mM EDTA; 0.5% NP-50; 150 mM KCl; 1 mM DTT; 35 mM Thiourea;
   b. briefly spin tubes containing substrate in a microcentrifuge before use.
6. Add and mix (by pipetting) 12 µl Nano-Glo^{®} Luciferase Assay Reagent per well
   - Wait approximately 3 minutes before measuring luminescence. The luminescence intensity will decay gradually, with a signal half-life of approximately 120 minutes at room temperature.
7. Measure luminescence in plate reader using standard protocol luciferase (this includes mixing sample in plate reader)

### Notes

Thaw Nano-Glo^{®} Luciferase Assay buffer to room temperature, but do not exceed 25°C at any moment during the procedure.

### Cell viability assessment with Resazurin assay

### Background:

This protocol is used to determine the metabolic activity of cells as a measure for cell viability after exposure to potentially toxic substances. Resazurin is converted in mitochondria by reduction to resorufin, which has a much higher fluorescence at ~600 nm compared to unconverted resazurin. A lower amount/percentage of conversion is correlated with reduced cellular metabolism and indirectly to cellular death and toxicity. Care must be taken with interpretation of the read-out, as 50% reduction in resazurin conversion may mean 50% reduction of cellular metabolism of 100% alive cells, or 50% of cells dead, and any combination in between.

### Procedure:

1. Prepare 0.25 mg/ml stock solution resazurin in fresh cell culture medium. Resazurin is auto-reducing over time in solution, especially in medium, and fresh stocks should be prepared every 2-3 days. Stocks should be stored at 4°C and protected from light. For HeLa cells, DMEM/F12 with 10% FCS is used.
2. Dilute stock solution 10x in fresh cell culture medium to 0.025 mg/ml -> ready for application on cells. For HeLa cells, DMEM/F12 with 10% FCS is used.
3. Collect 96-well plate and replace cell-culture spent medium with 100 µl per well resazurin solution. Add some resazurin solution for background measurement in wells not containing cells.
4. Incubate for **50 minutes** in incubator at 37°C, 5% CO₂, 95% RH.
5. Transfer resazurin solution to a fresh 96- or 384-well plate.
6. Measure in plate reader using 540/25 nm (excitation) and 610/40 nm (emission) using standard settings (PTM low/OD-1.0).
7. Subtract the average background measurement from all samples. Set the average of the negative controls (well receiving OptiMEM only during transfection) to 100% and express all values as percentage of the control activity. Typically, at lower levels of treatment, elevation of metabolic activity is shown, whereas for toxic substances, at toxic concentrations sigmoidal curves can be obtained, with lower activity indicating higher toxicity.

Results of the above experiments using lipids according to the invention as well as the reference compound (ALC-0315, CAS# 2036272-55-4) are summarized in Table 3 and 4 below.

**Table 3.**

| **Example** | **Size (nm)** | **PDI** | **Zeta potential (mV)** | **EE (%)** | **Transfection** | | |
|---|---|---|---|---|---|---|---|
| | | | | | **100 ng mRNA** | **50 ng mRNA** | **10 ng mRNA** |
| **1** | 226,7 | 0,223 | -10,74 | 53 | 1,69E+07 | 1,83E+07 | 3,81E+06 |
| **2** | 188,2 | 0,136 | -11,48 | 55 | 1,39E+07 | 1,60E+07 | 2,40E+06 |
| **3** | 185,7 | 0,122 | -9,667 | 77 | 1,37E+07 | 1,02E+07 | 9,69E+05 |
| **4** | 149,7 | 0,071 | -12,66 | 85 | 8,17E+06 | 1,23E+07 | 2,53E+06 |
| **5** | 291,8 | 0,169 | -9,117 | 96 | 6,52E+06 | 3,34E+06 | 4,17E+05 |
| **6** | 126,0 | 0,013 | -11,48 | 90 | 2,52E+07 | 1,83E+07 | 3,47E+06 |
| **7** | 152,7 | 0,022 | -15,85 | 92 | 2,01E+07 | 1,47E+07 | 1,62E+06 |
| **8** | 153,5 | 0,038 | -14,32 | 86 | 1,11E+07 | 1,36E+07 | 2,80E+06 |
| **9** | 167,3 | 0,144 | -13,37 | 67 | 1,43E+07 | 1,36E+07 | 2,79E+06 |
| **10** | 203,9 | 0,014 | -9,983 | 83 | 1,10E+07 | 3,57E+06 | 2,92E+05 |
| **ALC-0315 ref.** | 131,9 | 0,007 | -12,32 | 94 | 1,04E+07 | 1.47E+07 | 3,21E+06 |

**Table 4.**

| **Example** | **Cell Viability % (Average metabolic rate over all doses, Resazurin)** |
|---|---|
| **1** | 90 |
| **2** | 94 |
| **3** | 103 |
| **4** | 98 |
| **5** | 97 |
| **6** | 101 |
| **7** | 106 |
| **8** | 104 |
| **9** | 106 |
| **10** | 99 |
| **ALC-0315 ref.** | 96 |

### Conclusion

The sulphur atom incorporated in the tail sections of the lipids allowed to create a novel lipid library. Moreover, the thioether bond's susceptibility for oxidation is supposed to enhance the releasing of the cargo in the right place due to the formation of oxidized products (sulfoxide, sulfone) having polar function groups (SO, SO₂) that can destabilize the LNP. Based on the preliminary biological results, the lipids were capable for the formation of LNPs with appropriate particle size and polydispersity index (PDI) and have been shown to deliver Luc mRNA into cells effectively with very low cellular toxicity. Remarkably, some of the exemplified lipids exhibited higher transfection efficacy under these conditions compared to the reference compound ALC-0315.

## Claims

1. Compound of formula (I) 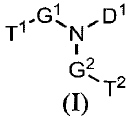 or its salt or stereoisomer,
wherein
G¹ is unsubstituted C₂-C₉ alkylene,
-(CH₂)ₓ-CH=CH-(CH₂)_{y}-, wherein x is an integer selected from 1 to 6, y is an integer selected from 1 to 6, and the sum of x+y is an integer selected from 2 to 7, or
-(CH₂)_{w}-X-(CH₂)_{z}-, wherein w is an integer selected from 1 to 7, z is an integer selected from 2 to 7, the sum of w+z is an integer selected from 3 to 8, wherein -(CH₂)_{z}- is attached to N, and X is selected from O, S;
T¹ is 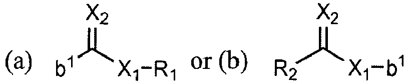 wherein
b¹ is a bond to G¹,
X₁ and X₂ are the same or different and each independently represents O or S,
R₁ is linear C₁-C₃₂ alkyl, branched C₃-C₃₂ alkyl, in both cases containing one S at any position in the carbon chain with the proviso that the heteroatom is not in the alpha or omega position of the carbon chain, or
R₁ is linear C₁-C₃₂ alkenyl, branched C₃-C₁₇ alkenyl in both cases containing one or more double bonds with the proviso that there is at least one -CH₂- group between the double bond and X₁ and in both cases containing one S at any position in the carbon chain with the proviso that the heteroatom is not in the alpha or omega position of the carbon chain and with the further proviso that there is at least one -CH₂- is between the heteroatom and the adjacent carbon atom of the double bond,
R₂ is linear C₁-C₃₂ alkyl, branched C₃-C₃₂ alkyl, in both cases optionally containing one S at any position in the carbon chain with the proviso that the heteroatom is not in the alpha or omega position of the carbon chain, or
R₂ is linear C₁-C₃₂ alkenyl, branched C₃-C₃₂ alkenyl containing one or more double bonds with the proviso that there is at least one -CH₂- group between the double bond and the carbon linking
X₁ and X₂ and in both cases containing one S at any position in the carbon chain with the proviso that the heteroatom is not in the alpha or omega position of the carbon chain and with the further proviso that there is at least one -CH₂- is between the heteroatom and the adjacent carbon atom of the double bond;
G² is defined as G¹ above, where G¹ and G² may be identical or different;
T² is defined as T¹ above, wherein b¹ is a bond to G², and where T¹ and T² may be identical or different;
D¹ is selected from 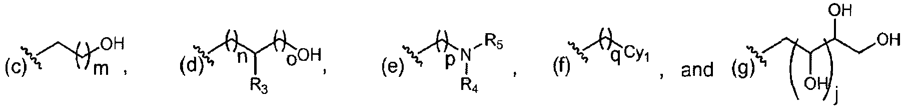 wherein
R₃ is C₁-C₆ alkyl, cyclopentyl, cyclohexyl, hydroxyl, hydroxymethyl, hydroxyethyl, phenyl, benzyl, 4-hydroxy benzyl, 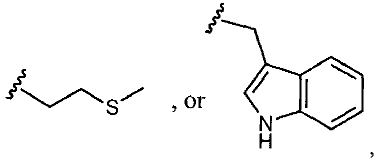
R₄ and R₅ are independently selected from H and C₁-C₆ alkyl,
m is an integer selected from 1 to 6,
n is an integer selected from 0 to 6,
o is an integer selected from 0 to 6,
p is an integer selected from 2 to 6,
q is an integer selected from 0 to 6,
j is an integer selected from 1 to 4,
Cy₁ is a C₃-C₆ cycloalkyl optionally substituted by one or more -OH, or
Cy₁ is a pyranose, furanose ring, which can be linked via any of its OH group, wherein the pyranose or furanose ring is optionally substituted by a -NH-CO-CH₃ group, adenine, guanine, uracil, cytosine, thymine, a mono- or oligosaccharide, or by a 4-, 5-, 6- or 7-membered heterocyclic ring containing 1, 2, 3 or 4 heteroatoms selected from N, O or S; or
Cy₁ is a 4-, 5-, 6- or 7-membered heterocyclic ring containing 1, 2, 3 or 4 heteroatoms selected from O, N or S, wherein the heterocyclic ring is optionally substituted by R₆,
wherein
R₆ is C₁-C₆ alkyl, an arginine containing peptide, a pyranose or furanose ring attached by any of their ring-carbon atoms to the 4-, 5-, 6- or 7-membered heterocyclic ring, wherein the pyranose or furanose ring is optionally substituted by a -NH-CO-CH₃ group, adenine, guanine, uracil, cytosine, thymine or a mono- or oligosaccharide, or
R₆ is a group selected from (c), (d), (e) or (g), wherein, m, n, o, p, j, R₃, R₄ and R₅ are as defined above, or
R₆ is a 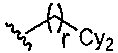 group wherein r is an integer selected from 1 to 4, and Cy₂ is a 4-, 5-, 6- or 7-membered heterocyclic ring containing 1, 2, 3 or 4 heteroatoms selected from O, N and S, optionally substituted by C₁-C₆ alkyl.

2. Compound according to claim 1, wherein G¹ and G² are the same or different and each is independently linear C₄-C₉ alkylene, preferably C₅-C₉ alkylene, or, alternatively, each is independently linear C₅, C₆, C₇, or C₉ alkylene.

3. Compound according to claim 1 or 2 wherein G¹-T¹ and G²-T² are identical.

4. Compound according to any of claims 1 to 3 wherein T¹ and/or T² is selected from the following:

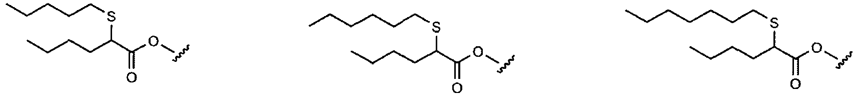

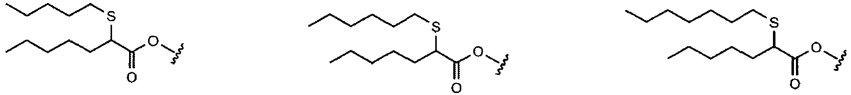

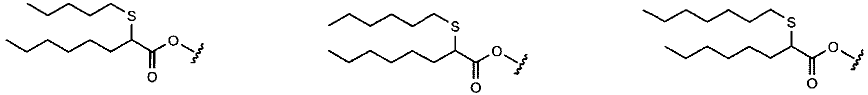

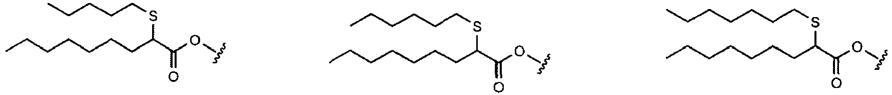

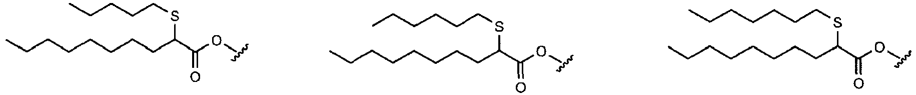

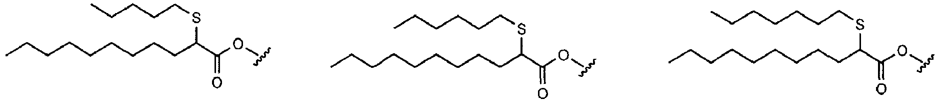

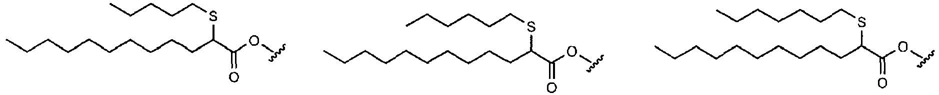

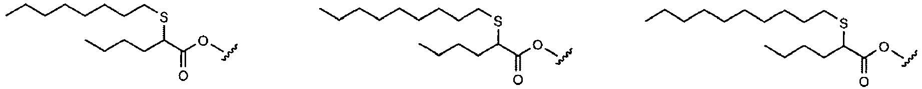

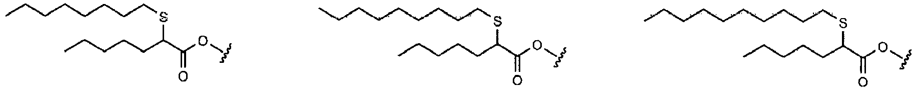

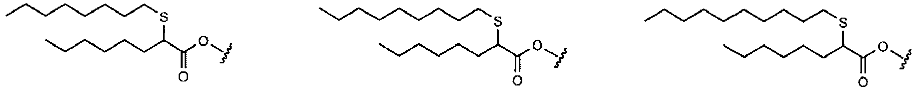

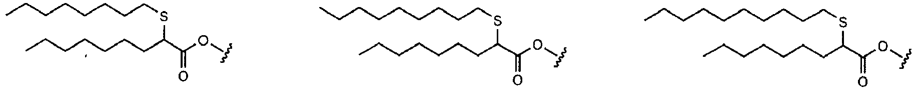

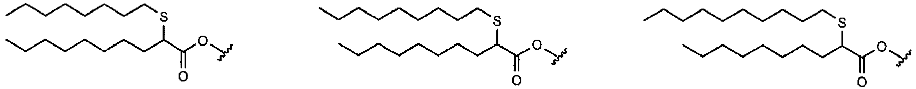

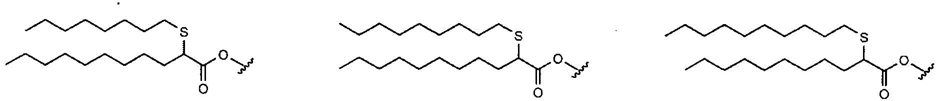

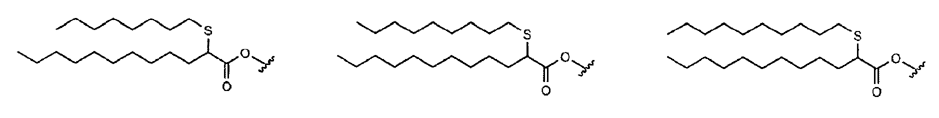

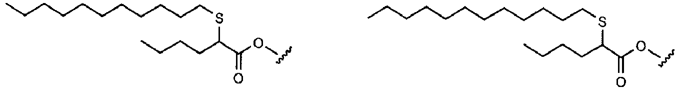

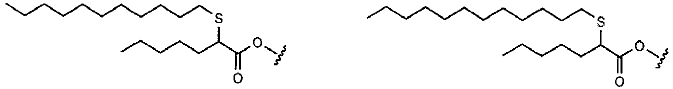

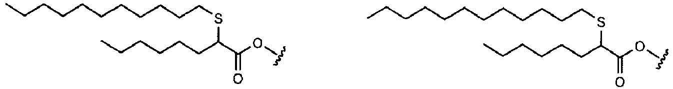

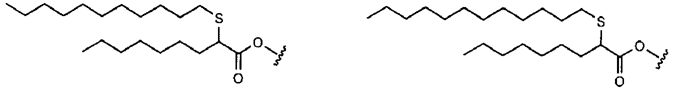

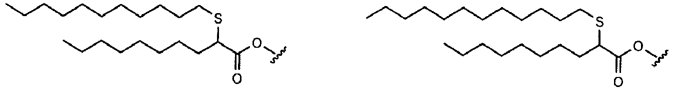

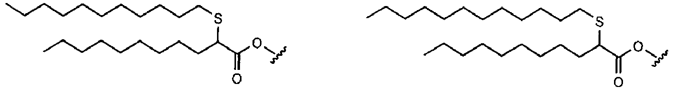

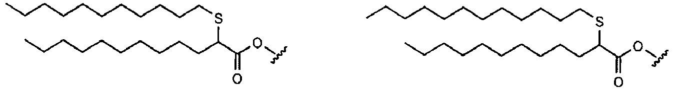

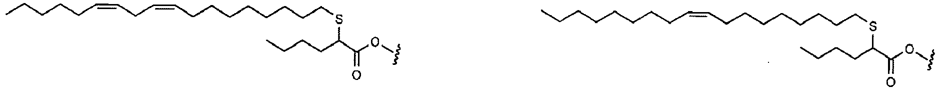

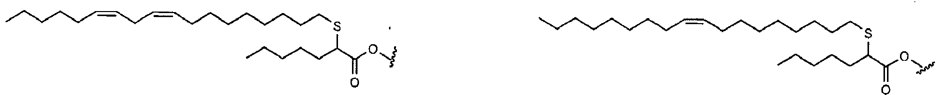

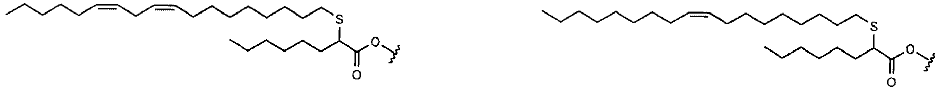

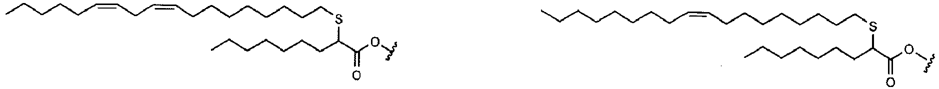

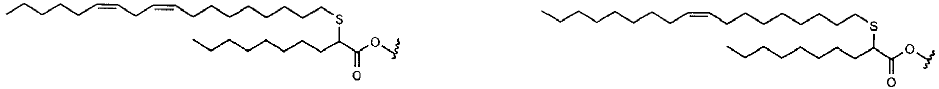

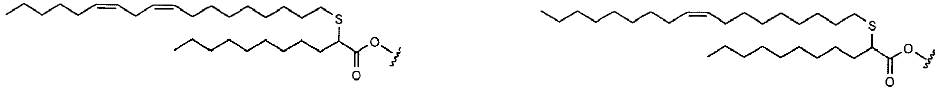

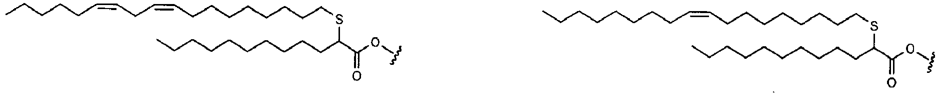

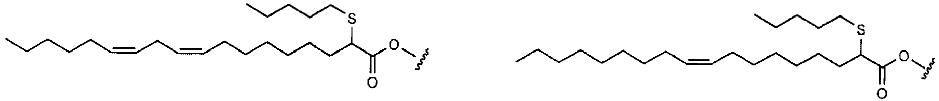

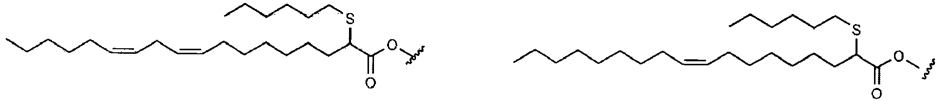

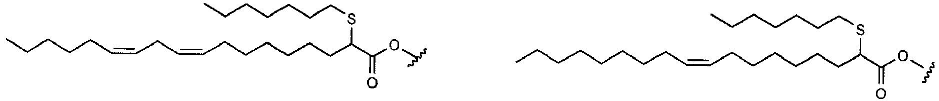

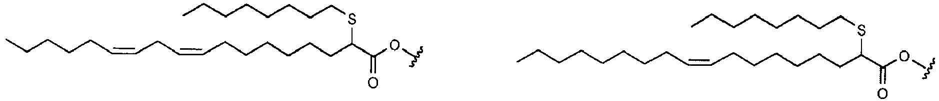

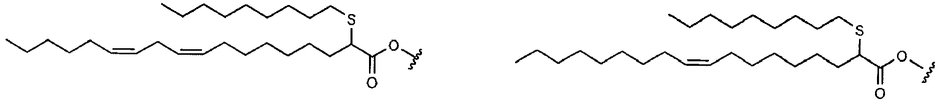

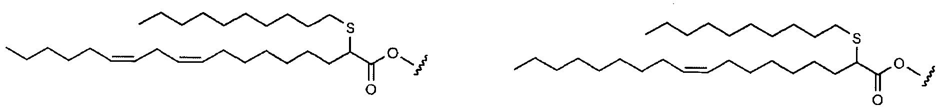

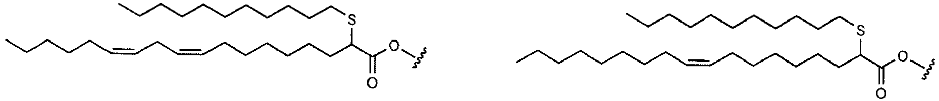

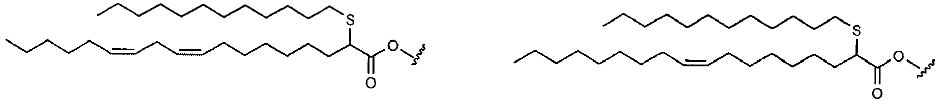

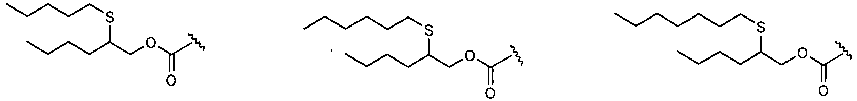

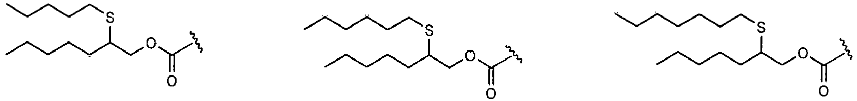

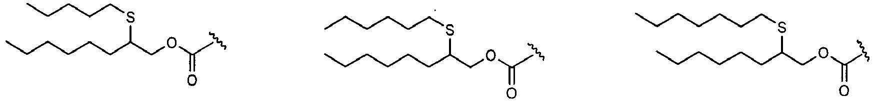

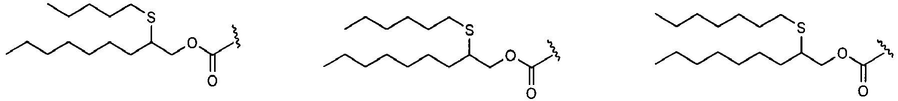

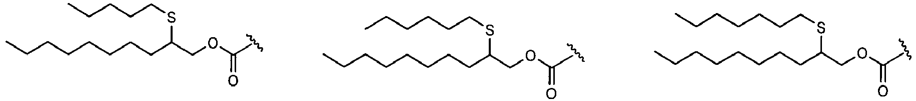

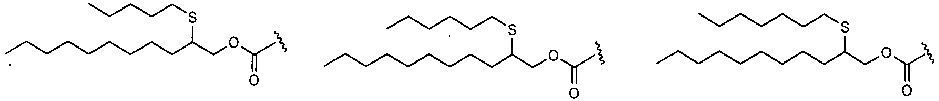

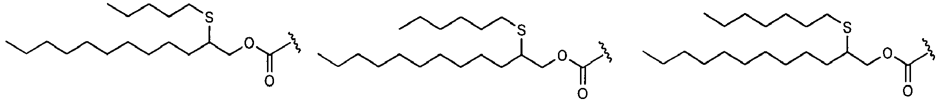

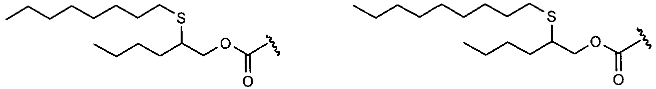

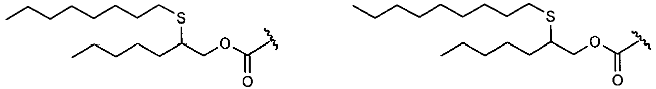

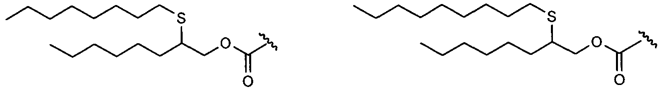

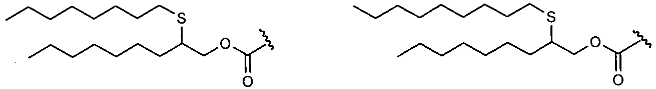

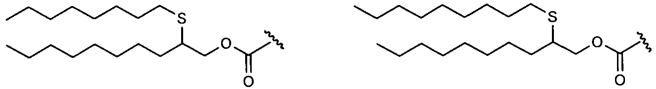

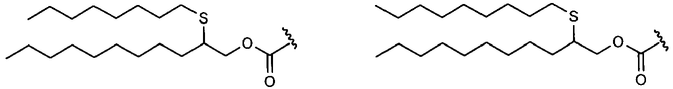

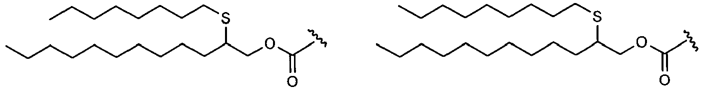

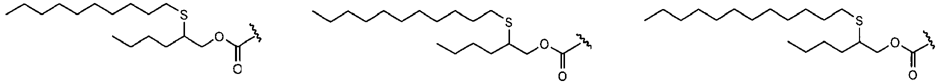

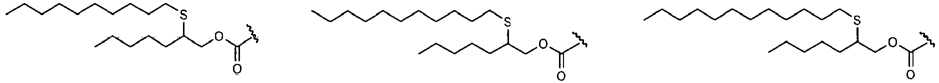

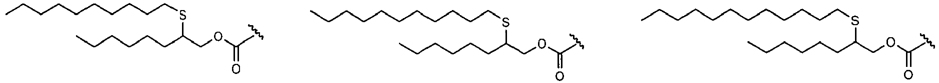

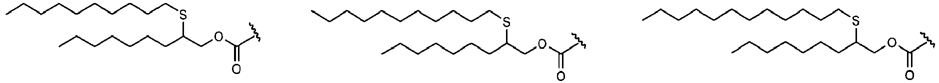

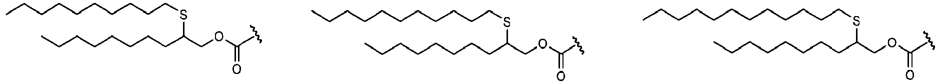

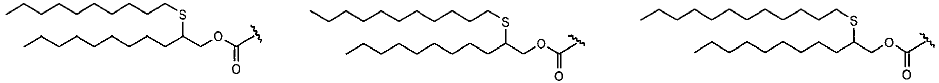

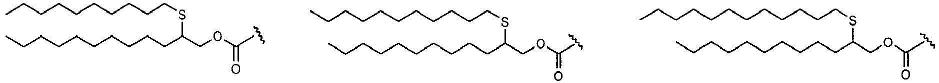

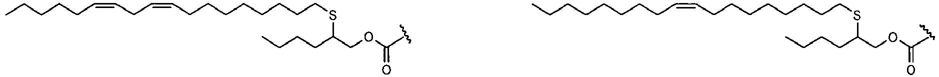

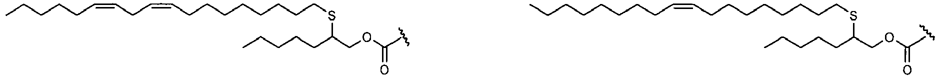

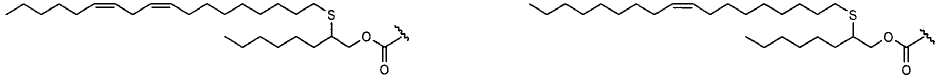

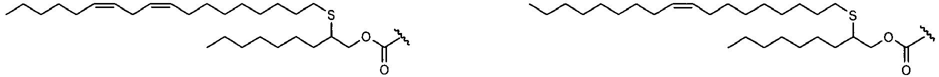

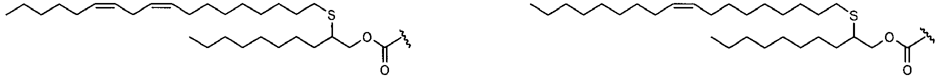

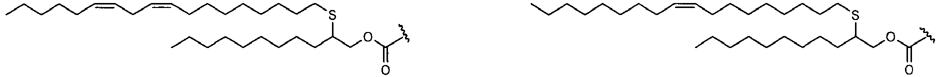

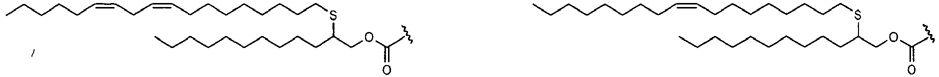

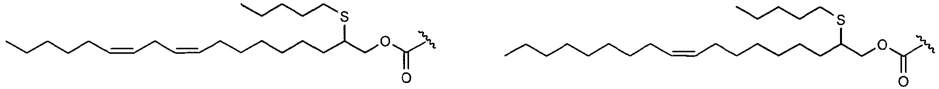

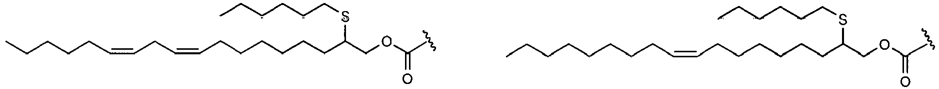

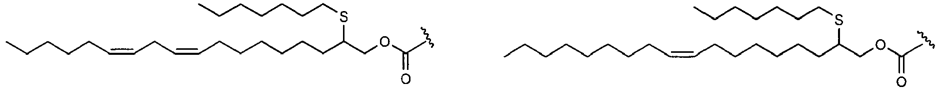

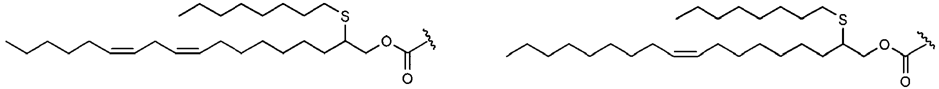

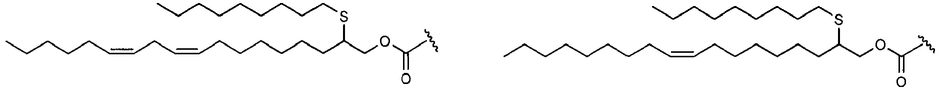

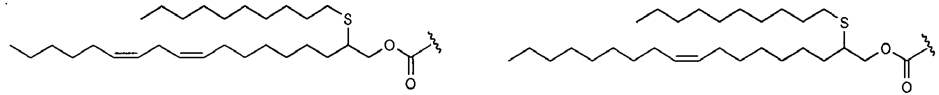

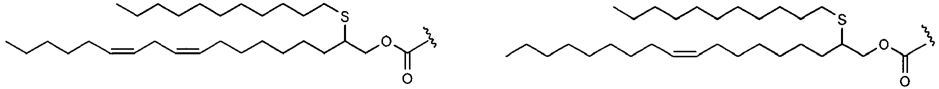

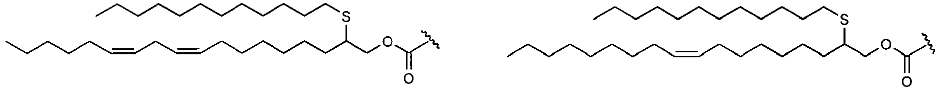

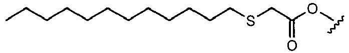

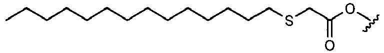

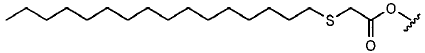

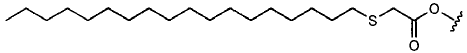

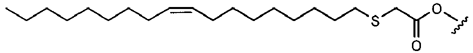

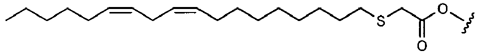

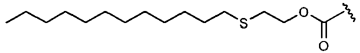

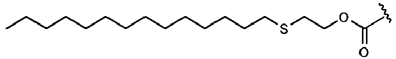

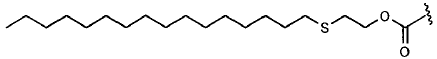

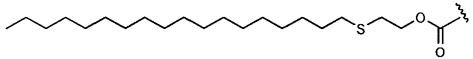

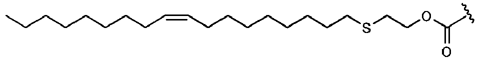

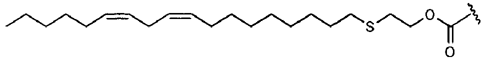

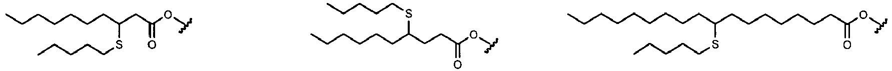

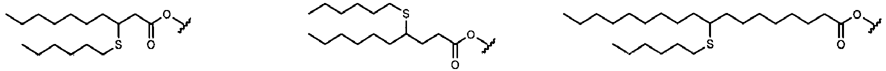

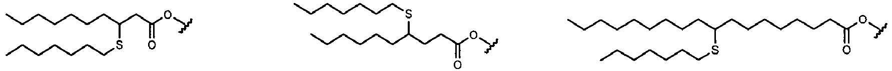

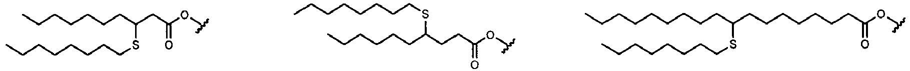

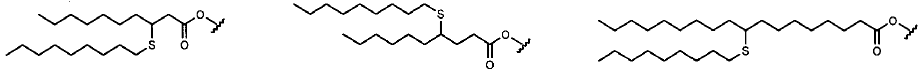

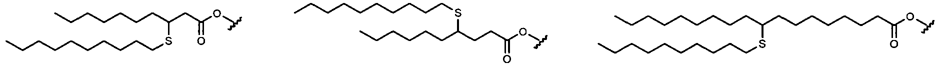

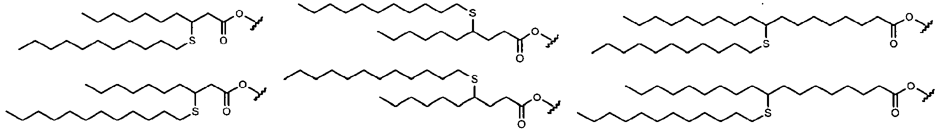

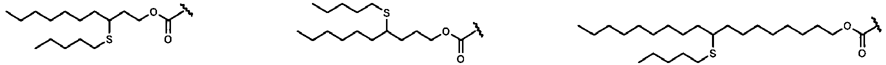

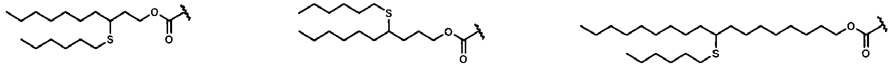

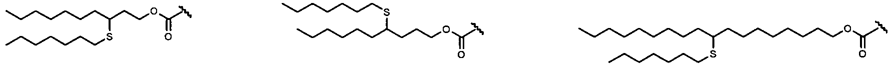

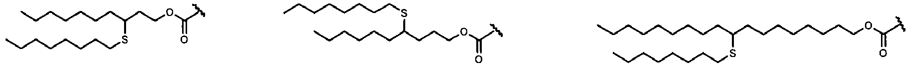

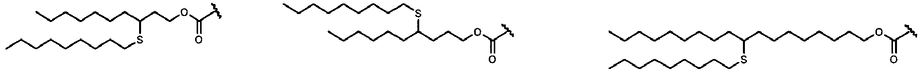

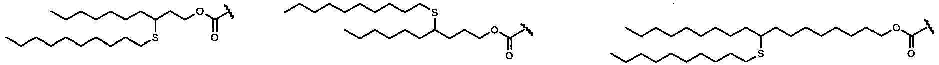

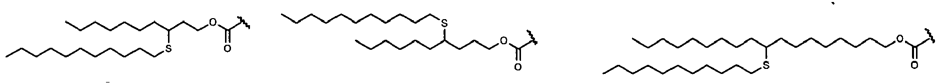

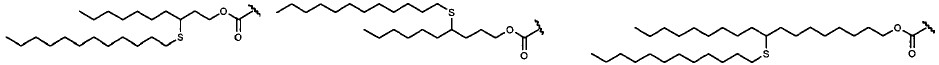

5. Compound according to any of claims 1-4, wherein T¹ and/or T² is (b).

6. Compound according to claim 5 wherein in R₂ the heteroatom S is in the beta position of the carbon chain.

7. Compound according to any of claims 1 to 6 wherein R₁ and/or R₂ is selected from the following:

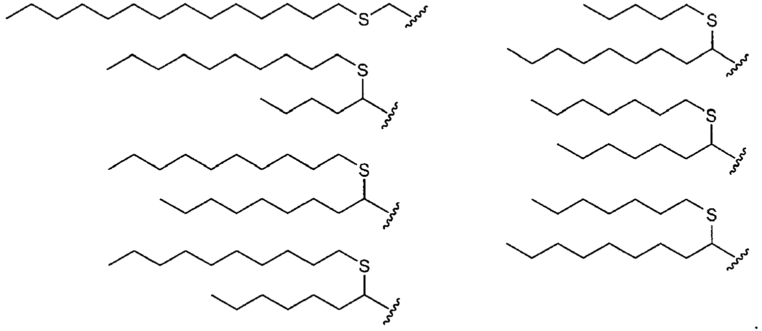

8. Compound according to any of claims 1 to 7 wherein D¹ is selected from the following: 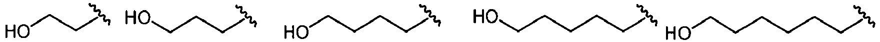 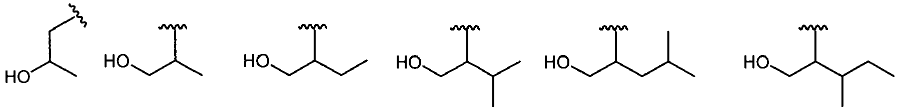 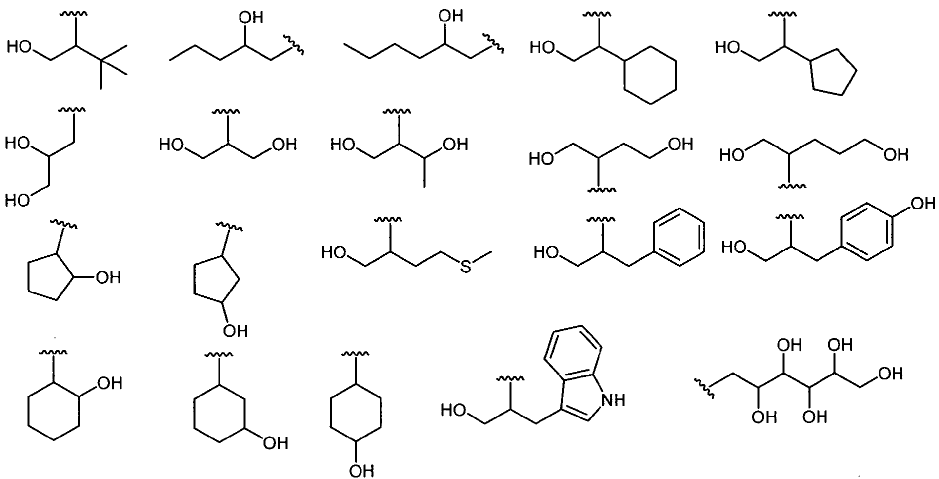 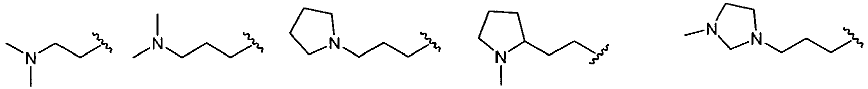 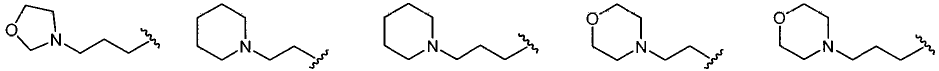 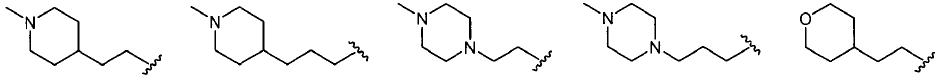 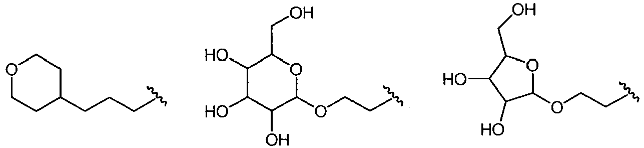 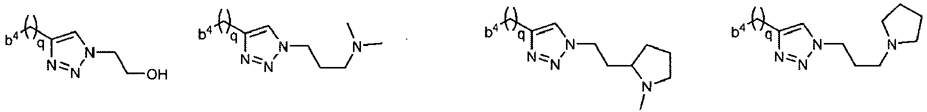 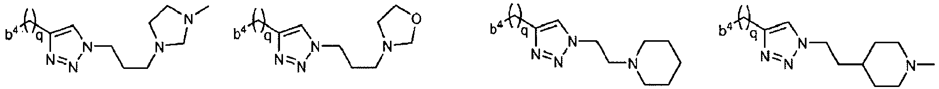 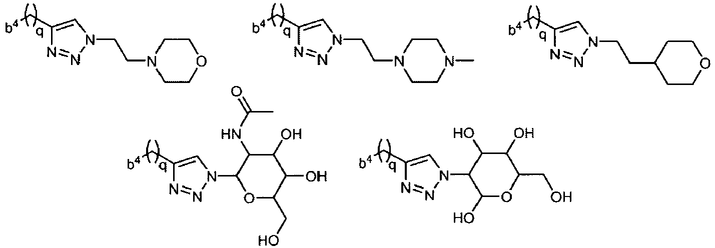 wherein q is an integer selected from 1 to 4 and b⁴ is a bond to the nitrogen.

9. Compound according to claim 8 wherein D¹ is

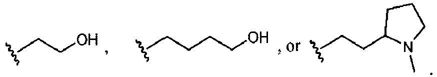

10. Compound according to claim 1 which is selected from the following:
